# EUROPEAN PATENT APPLICATION

(11) **EP 2 684 570 A1**
(43) Date of publication of application: **15.01.2014**
(21) Application number: 12178248.6
(22) Date of filing: 09.09.2009
(51) Int. Cl.: A61K 38/29, A61K 47/26

(54) **Compositions and methods for the prevention of oxidative degradation of proteins**

(30) Priority: 10.09.2008 US 95878 P
(62) Divisional of application: 09792371.8
(71) Applicant: F. Hoffmann-La Roche AG, 4070 Basel (CH)
(72) Inventor: Ji, Junyan A., Foster City, CA California 94404 (US); Wang, Yuchang John, Los Altos, CA California 94022 (US); Zhang, Boyan, Foster City, CA California 94404 (US)
(74) Representative: Brodbeck, Michel

(57) **Abstract**

The invention relates to pharmaceutical formulations comprising a protein and free methionine in combination with one or more vitamins or vitamin derivatives capable of preventing the oxidation of aromatic amino acid residues within a protein. More specifically, the invention relates to stabilized, pharmaceutically effective preparations of oxidation-sensitive therapeutic agents. The invention further relates to a method of inhibiting the oxidation of such therapeutic agents.

## Description

### FIELD OF THE INVENTION

The invention relates to the use of aromatic compounds as stabilizers to prevent oxidative degradation of proteins. More specifically, the invention relates to stabilized, pharmaceutically effective preparations of oxidation-sensitive therapeutic agents. The invention further relates to a method of inhibiting the oxidation of such therapeutic agents.

### BACKGROUND OF THE INVENTION

Proteins undergo varying degrees of degradation during purification and storage. Oxidation is one of the major degradation pathways of proteins, and has a destructive effect on protein stability and potency. Oxidative reactions cause destruction of amino acid residues, peptide bond hydrolysis, and hence protein instability due to alteration of the protein's tertiary structure and protein aggregation (Davies, J. Biol. Chem. 262: 9895-901 (1987)). Oxidation of protein pharmaceuticals have been reviewed by Nguyen (Chapter 4 in Formulation and Delivery of Protein and Peptides (1994)), Hovorka, (J. Pharm Sci. 90:25369 (2001)) and Li (Biotech Bioengineering 48:490-500 (1995)).

### Causes of Protein Oxidation

Oxidation occurs via many different and interconnected pathways, and is catalyzed by a variety of triggering conditions, including elevated temperature, oxygen levels, hydrogen ion levels (pH), and exposure to transition metals, peroxides and light. Typically, a significant factor causing oxidative degradation of proteins is exposure to oxygen, reactive oxygen species and metals. Certain excipients are formulated in pharmaceutical compositions to provide protection against protein aggregation, but these agents can also enhance oxidation because they contain reactive oxygen species. For example, commonly used surfactants, such as Polysorbate 80 (commonly known as Tween), contain trace amounts of peroxide contaminants, which can cause further oxidation of the surfactant to generate greater amounts of reactive oxygen species (oxygen radicals) in the presence of low concentrations of metals (Ha et al., J Pharm Sci 91:2252-2264 (2002); Harmon et al., J Pharm Sci 95:2014-2028 (2006)). The combination of the oxygen radicals and metals thereby provides a catalytic environment for the oxidation and, thus, degradation of the protein formulated with the surfactant. Oxidation of proteins in liquid or lyophilized formulations is also shown to be triggered by the peroxide in polysorbates or other formulation excipients such as polyethylene glycols (PEG) and trace amounts of metal such as iron or copper. In addition, pharmaceutical preparations commonly are packaged in plastic containers made of low density polyethylene (LDPE) or polypropylene for convenient storage and application. However, these plastic containers are readily permeable to oxygen. The oxygen forms reactive oxygen species which cause rapid oxidation of the oxidation-sensitive residue(s) in the pharmaceutical protein, such as the oxidation of methionine to methionine sulfoxide. (Manning et al., Pharmaceutical Research, Vol. 6, No. 11, (1989)).

It is the side chains of Cysteine (Cys), Methionine (Met), Tryptophan (Trp), Histidine (His), and Tyrosine (Tyr) residues which are particularly vulnerable to oxidation, in order of sensitivity. The sensitivity of these amino acid residues to oxidation is a result of adduct species formed with aromatic rings which are stabilized by delocalization on to neighboring double bonds. The thiol group in Cys is the most reactive functional group, because the thiol group offers ready hydrogen extraction by the radicals, and for that reason very few pharmaceutical proteins contain free Cys.

### Methionine oxidation forms Met sulfoxide (Met[O]).

Methionine oxidation forms Met sulfoxide (Met[O]) and, under extreme conditions, sulfone. The following examples represent pharmaceutical proteins exhibiting Met oxidation and the oxidants used in each study are identified: growth hormone (hGH, Teh, L-C, J. Biol Chem 262:6472-7, (1987) using H₂O₂, Pearlman R, Chapter 1, Pharmaceutical Biotechnology vol 5 (1993), Zhao F, J. Biol Chem 272:9019-9029 (1997), using Asc/Cu(II)/ O₂), IL-2 (Sasaoki K, Chem Pharm Bull 37:2160-4 (1989) using 100x fold H₂O₂, Cadé JA, Pharm Res. 18:1461-7 (2001) using peroxodisulfate, Ha E, J Pharm Sci 91:2252-64, (2002) using Tween), small peptides (Li, Pharm Res 12: 348-55 (1995)), relaxin (Nguyen TH, Pharm Res. 10:1563-71 (1993) and Chapter 5 in Pharmaceutical Biotechnology vol 9 (1996) using 2000x H₂O₂, Li, Biochem 34: 5762-72 (1995) using Asc/Cu(II)/ O₂), rhGCSF (Lu HS, Arch Biochem Biophys 362:1-11 (1999), Herman AC, Chapter 7 in Pharmaceutical Biotechnology vol 9 (1996), Yin, Pharm Res 21: 2377-83 (2004) and Pharm Res 22: 141-7 (2005) using H₂O₂), rhVEGF (Duenas ET, Pharm Res. 18:1455-60 (2001), using H₂O₂ & tBHP), IGF-1 (Fransson, Pharm Res 13:1252 (1996), using dissolved O₂, Fe(III) EDTA), rhCNF and rhNGF (Knepp V, PDA J Pharm Sci Tech. 50:163-171 (1996), using H₂O₂), BDNF (Jensen JL, Pharm Res. 17:190-6 (2000), using Asc/Cu(II)/ O₂), rhLeptin (Liu JL, Pharm Res. 15:632-40 (1998) using tBHP and H₂O₂), Actimmune and Activase (Keck RG, Anal Biochem. 236, 56-62 (1996), using tBHP), Herceptin (Shen FJ, Techniq. Protein Chem. VII. 275-284 (1996) using tBHP, Lam XM, J Pharm Sci 86:1250-5 (1997) using thermal, light and stainless steel), and PTH (Yin et al., Pharm Res 22:141-7 (2004), Chu et al., Biochem 43: 14139-48 (2004) and (Chu et al., J Pharm Sci 93:3096-102 (2004), using H₂O₂), and a monoclonal antibody (Wei et al. Anal Chem. 79: 2797-805, 2007; using tBHP, UV irradiation and ozone). It is noteworthy that in the past 20 years, a great variety of oxidants have been used to study the oxidation of proteins. tBHP and H₂O₂ have been used predominantly. Except for ascorbate, all were metal free oxidants.

### Histidine oxidation forms oxo-histidine.

His oxidation predominantly forms oxo-histidine but also forms a variety of other oxidation products, depending on the oxidation conditions. By using Asc/Cu(II)/O₂, Li et al. (J Pharm Sci. 85:868-72, 1996) observed oxidation of the His residues in relaxin. With human growth hormone, Zhao et al. (J Biol Chem 272:9019-9029, 1997) observed oxo-histidine when the same oxidizing system was used to simulate metal-catalyzed oxidation at the metal-binding site. Aspartic acid and asparagine as oxidation products of His were also detected in β-amyloid peptide in the presence of Cu(II)/H₂O₂ (Kowalik-Jankowska et al., J Inorg Biochem 98 (6):940-950,2004).

### Tryptophan oxidation.

With respect of tryptophan oxidation, multiple products are formed. Stability studies of tryptophan in aqueous solution (Lee, J Parent Sci Tech 42: 20-2 (1988)) and tryptophan residues in small peptides and lysozyme (Simat TJ, J Agric Food Chem 46:490-8 (1998)) and in bovine α-crystallin (Finley EL, Protein Sci 7:2391-7 (1998)) clearly identified the main degradants as being 5-hydroxy-tryptophan, oxy-indole alanine, kynurenine and N-formylkynurenine. Compared with the other forms of amino acid oxidation, there are relatively few articles on the oxidation of Trp in pharmaceutical proteins. Davies et al. (J Biol Chem. 262: 9902-7, 1987) oxidized bovine serum albumin by oxygen radicals generated from cobalt radiation, Uchida et al. (Agric Biol Chem 53:3285-92, 1989) stressed albumin with Fe(II)/EDTA/Asc and detected selective oxidation of Trp and His. Recently, Trp oxidation in monoclonal antibodies was reported by Amgen (Yang et al. J Chrom. A. 1156: 174-82, 2007) and MedImmune (Wei et al. Anal Chem. 79: 2797-805, 2007) stressed by ozone and UV irradiation). At Genentech, Trp oxidation has been detected in anti-VEGF, anti-CD40, anti-CD22 and Apomab antibodies. With Apomab, declining potency was correlated with the degree of Trp oxidation.

### Mechanisms of oxidation

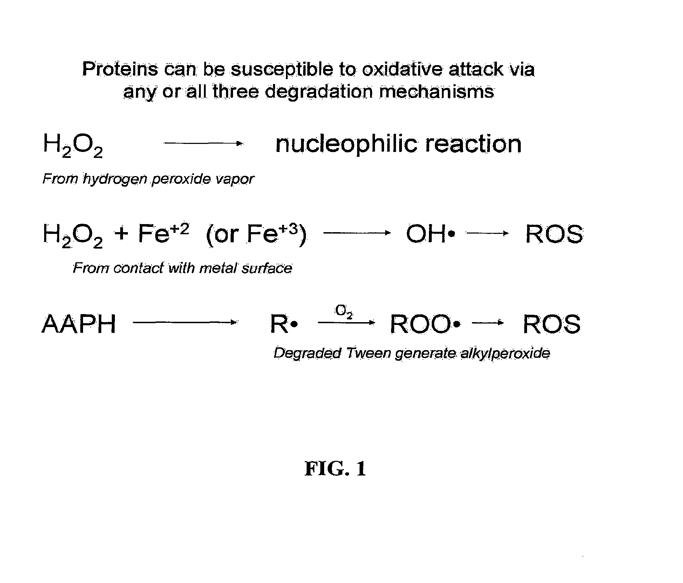

Based on the analyses above, proteins can be susceptible to oxidative attack via any or all three degradation mechanisms shown in Figure 1, as well as light-induced oxidation. The nucleophilic reaction with H₂O₂ can be the oxidation reaction observed when protein product is exposed to vapor H₂O₂ used as aseptic agent in protein isolators, or from the degradation of commonly used excipients, such as polysorbates (e.g. Tween) or polyethylene glycols. When trace metal (iron, copper, or chromium) is brought to the formulation solution, for example from contact with stainless steel, a Fenton reaction, H₂O₂ with Fe(II), becomes operative. A third degradation mechanism is via alkylperoxides which could come from degraded Tween, as described above. (Jaeger J, J Biochem Biophys Methods 29: 77-81, 1994).

Protein pharmaceuticals subject to oxidation often results in modification of the protein and potency loss. Oxidation of proteins such as monoclonal antibody-containing solutions can result in degradation, aggregation and fragmentation of the antibody, and thus loss of antibody activity. In other cases, even though the protein pharmaceutical is still biologically active after oxidation, the growth factor may not be acceptable for pharmaceutical use according to the standards of regulatory agencies, such as the FDA, for example, when high levels of methionine sulfoxide are present. Current precautionary procedures to exclude oxygen during the manufacture and packaging of the preparation have proven to be ineffective in preventing significant oxidation of pharmaceutical proteins. The result is that the pharmaceutical preparation has a shorter effective life than is potentially possible if the oxidation reaction could be inhibited. Thus, there is a need in the art to identify physical and chemical conditions that will remedy the acceleration of protein degradation, in order to provide stable protein-containing pharmaceutical compositions that can endure oxidative conditions over a period of time. It is therefore desirable to formulate peptide- and antibody-containing pharmaceutical compositions with excipients that will protect proteins from oxidative damage due to a variety triggering factors.

### Oxidation Stabilizers in the Art

Certain amino acids, and various combinations thereof, along with surfactants, such as polysorbate and poloxamer and the like, have been used to stabilize peptide and protein compositions. See, for example, Yu-Chang John Wang and Musetta A. Hansen, "Parenteral Formulations of Proteins and Peptides: Stability and Stabilizers", Journal of Parenteral Science and Technology, 42:S14, 1988. A collection of antioxidants for all injectable products was compiled by Neema et al. (J Pharm Sci Tech 51: 166-71(1997)) where bisulfite, ascorbate, butylated hydroxyl anisole, cysteine, etc. were listed. However, none of the listed agent appears effective in dealing with the entire spectrum of oxidative degradation mechanisms and the concomitant oxidation of not only methionine but also tryptophan and other aromatic amino acid residues in a protein. As an antioxidant, free methionine was first cited in US Patent 5,272,135 (Takruri, 1993) and also in a US Patent Application 2003/0104996 A1 (Li, 2003). Free methionine can be found in a number of marketed parenteral products such as: depo-subQ Provera, Follistim AQ, Gonal-f RFF, Lutropin-α. Histidine has also been disclosed as a potential antioxidant in US Patent 5,849,700 (Sorensen et al., 1998). Sorensen et al. disclose that a pharmaceutical preparation comprising a growth hormone and histidine or a derivative of histidine as additive or buffering substance demonstrated a very high stability against deamidation, oxidation (as measured by sulfoxide concentrations) and cleavage of peptide bonds. Other agents that may control the oxidation of protein include metal chelating agents (e.g. EDTA) and free radical scavengers (e.g. mannitol), which have been widely cited in textbooks and review articles. See, for example, Yu-Chang John Wang and Musetta A. Hansen, "Parenteral Formulations of Proteins and Peptides: Stability and Stabilizers", Journal of Parenteral Science and Technology, 42:S14, 1988. N-acetyl tryptophanate has been used along with octanoate as a ligand that binds to specific sites to stabilize human serum albumin during pasteurization (Peters Biochemistry, genetics, and medical applications. Academic Press, NY, 1995). However, none of the amino acids or surfactants are used to deter oxidation via alkylperoxides which could come from degraded surfactants. Therefore, there is a need for a method of inhibiting multiple mechanisms of oxidation in pharmaceutical vehicles of polypeptides having an amino acid sequences susceptible to oxidative attack.

### SUMMARY OF THE INVENTION

The present invention provides improved compositions and methods for protecting proteins against damage due to oxidation. The compositions contain one or more proteins susceptible to oxidation formulated together with one or more compounds capable of effectively curtailing the free radical mediated oxidation that typically causes tryptophan, tyrosine or histidine residues to oxidize. The compositions exhibit increased resistance from oxidation resulting in, for example, a longer product shelf life, greater stability allowing room temperature storage, and/or greater flexibility in product packaging. Accordingly, the present invention provides an important means for protecting (i.e., stabilizing) even multi-unit protein compositions, such as antibody compositions.

In one embodiment of the present invention, a pharmaceutical formulation is provided comprising a protein formulated (e.g., in a preparation, such as a laboratory-grade or pharmaceutical composition) with one or more compounds capable of preventing the oxidation of aromatic amino acid residues within said protein. Preferred embodiments utilize free aromatic amino acids, nucleotides or vitamins and their derivatives in combination with methionine, which together effectively protect against all of the most common mechanisms of protein oxidation.

In another embodiment of the present invention, a method is provided for preparing a stabilized protein composition by formulating a protein together with methionine in combination with one or more compounds capable of preventing the oxidation of aromatic amino acid residues within said protein as described below.

In another embodiment of the present invention, a method is provided for preventing or treating a disease or disorder in a mammal comprising administering the formulation comprising a protein-based therapeutic agent and one or more compounds capable of preventing the oxidation of aromatic amino acid residues within said agent to the mammal in an amount effective to prevent or treat said disease or disorder.

In another embodiment of the present invention, a method of making a pharmaceutical formulation is provided comprising preparing the formulation comprising a protein and one or more compounds capable of preventing the oxidation of aromatic amino acid residues within said protein and evaluating physical stability, chemical stability, or biological activity of the protein in the formulation.

In another embodiment of the present invention, a method is provided for stabilizing a pharmaceutical composition of a protein which comprises adding methionine and one or more compounds to said composition in an amount sufficient to inhibit oxidation of aromatic amino acid residues within said protein.

In another embodiment of the present invention, a method is provided for making a pharmaceutical formulation comprising adding an amount of a surfactant to a protein composition and an amount of a compound sufficient to negate the oxidative species generated from the degradation of said surfactant.

In another embodiment of the present invention, a method is provided for preventing the oxidation of aromatic amino acid residues within a susceptible protein which comprises adding methionine in combination with one or more compounds selected from the group consisting of aromatic amino acids, nucleotides, vitamins and their derivatives.

Other features and advantages of the invention will be apparent from the following detailed description and examples which should not be construed as limiting. The contents of all references, patents and published patent applications cited throughout this application are expressly incorporated herein by reference.

### BRIEF DESCRIPTION OF THE DRAWINGS

Figure 1. A scheme depicting possible routes for oxidation of methionine, tryptophan and histidine.
Figure 2. A scheme depicting AIBN, an azo compound that generates alkyl radical upon heating, when combined with oxygen forms alkylperoxide (circled). AAPH, another azo compound, is also shown with its structure.
Figure 3. An illustration of the rp-HPLC chromatogram of PTH degraded by H₂O₂. Reaction at 40°C, and samples were removed at 2, 4, and 6 hours. Increased peak height of monooxidized PTH and dioxidized PTH is shown.
Figure 4. A chromatogram of PTH treated with AAPH, predominantly Trp[O]-PTH peaks are shown. Reaction at 40°C, and samples were removed at 2, 4, and 6 hours.
Figure 5. Chemical structures of the degraded (oxidized) tryptophan. Their masses are noted, as +4, +16 and +32.
Figure 6. rpHPLC chromatogram of PTH solution oxidized by AAPH, H₂O₂ plus iron, and H₂O₂. Reaction conducted at 40°C, 6 hours. Samples were either with or without the addition of free methionine 2mg/mL.
Figure 7. rpHPLC chromatogram of PTH solution oxidized by AAPH, H₂O₂ plus iron, and H₂O₂. Reaction conducted at 40°C, 6 hours. Samples were either without or with the addition of 15% mannitol or 6% sucrose.
Figure 8. rpHPLC chromatogram of PTH solution oxidized by AAPH, H₂O₂ plus iron, and H₂O₂. Reaction conducted at 40°C, 6 hours. Samples were either with or without the addition of EDTA mg/mL.
Figure 9. rpHPLC chromatogram of PTH solution oxidized by AAPH, H₂O₂ plus iron, and H₂O₂. Reaction conducted at 40°C, 6 hours. Samples were either with or without the addition of free tryptophan at 2mg/mL.
Figure 10. rpHPLC chromatogram of PTH solution oxidized by AAPH, H₂O₂ plus iron, and H₂O₂. Reaction conducted at 40°C, 6 hours. Samples were either with or without the addition of free tryptophan AND methionine both at 2mg/mL.
Figure 11. Graph of data showing site specific oxidation of PTH by AAPH and the different protection roles of Trp and Met comparing with other reagents. The identification of oxidation of individual Trp23, Met8 and Met 18 residues was assigned based on the MS/MS fragmentation spectra of their corresponding tryptic peptides. The relative oxidation level was quantified based on the integrated extracted ion chromatograms of oxidized and non-oxidized peptides.
Figure 12. Graph of data showing site specific oxidation of PTH by H2O2/Fe and the different protection roles of Trp and Met comparing with other reagents. The identification of oxidation of individual Trp23, Met8 and Met18 residues was assigned based on the MS/MS fragmentation spectra of their corresponding tryptic peptides. The relative oxidation level was quantified based on the integrated extracted ion chromatograms of oxidized and non-oxidized peptides.
Figure 13. IEC chromatogram of various anti-VEGF samples. H2O2 generated no oxidative species in basic region, AAPH did. Basic peaks were prominent in qualification lot when a bad lot of Tween was used.
Figure 14. IEC of anti-VEGF antibody when oxidized by AAPH (no Trp), then 2 or 10 mg/mL free Trp was added to the formulation. Oxidized MAb eluted in basic region. These basic peaks dropped to the baseline upon addition of Trp.
Figure 15. rpHPLC chromatogram of PTH solution oxidized by AAPH, H₂O₂ plus iron, and H₂O₂. Reaction conducted at 40°C, 6 hours. Samples were either with or without the addition of free Trolox (6-hydroxy-2,5,7,8-tetramethylchroman-2-carboxylic acid, a water soluble vitamin derivative) at 2mg/mL.
Figure 16. rpHPLC chromatogram of PTH solution oxidized by AAPH, H₂O₂ plus iron, and H₂O₂. Reaction conducted at 40°C, 6 hours. Samples were either with or without the addition of free pyridoxine (commonly known as vitamin B6) at 2mg/mL.

### DETAILED DESCRIPTION OF THE PREFERRED EMBODIMENT

### I. Definitions

The chemical instability of proteins can involve the cleavage or formation of covalent bonds with the protein primary structure. Several oxidation reactions in proteins have been reported. In the alkaline or neutral medium the residues of the amino acids cysteine, histidine, methionine, tryptophan and tyrosine are especially prone to oxidation. In acidic conditions, however, methionine is sensitive. Often the oxidation reactions cause a great loss in biological activity and even immunogenicity. The present invention relates primarily to improved compositions and methods for protecting proteins against damage due to oxidation. The compositions contain one or more proteins susceptible to oxidation formulated together with one or more aromatic compounds to effectively curtail free radical mediated oxidation that typically causes tryptophan, tyrosine or histidine residues to oxidize.

Because aromaticity (as exemplified in the aromatic rings of purines and pyrimidine in nucleotides or, specifically, indole in the amino acid tryptophan) can delocalize the extra electron when an aromatic compound reacts with a free radical, the product is stabilized by electron delocalization. Consequently, the reaction between aromatic compounds and free radicals is favored. The net result is that the free radical is absorbed into the aromatic compound, and unable to do further damage to other molecules. For this reason, aromatic compounds, when added as formulation excipients, serve as effective agents to neutralize the oxidative damaging effects of free radicals.

Two major classes of aromatic compounds that are physiologically compatible are nucleotides and amino acids. As these compounds are natural components of body chemistry, they have conducive safety profiles and are suitable for use as excipients for parenteral products. Free methionine has been routinely used as an antioxidant and can be found in a number of marketed parenteral products. However, this amino acid alone does not protect against all mechanisms of oxidation and is most effective in inhibiting nucleophilic oxidation of methionine or cysteine residues. It is also well known that DNA is highly susceptible to damage by free radicals, a fact that supports the use of nucleic acid derivatives to react favorably with free radicals. To date, little is known about using nucleic acid derivative as formulation excipients and no product on the market utilizes nucleic acid as formulation excipients.

In one aspect of the invention, compositions of the present invention typically contain aromatic amino acid selected from the group consisting of tryptophan, histidine, tyrosine and phenylalanine. The preferred aromatic amino acid for mitigating oxidation via alkylperoxides, which are often generated from degraded surfactants, is tryptophan or its derivative, sodium N-acetyl tryptophanate. When methionine or methionine derivatives are added to the formulation, nucleophilic oxidation of methionine or cysteine can also be inhibited. Thus, a combination of free tryptophan and methionine effectively inhibits multiple mechanisms of oxidation. Compositions wherein the tryptophan is present in the formulation typically contain an amount ranging from about 2 - 10 mg/ml. In one embodiment, the invention relates to a pharmaceutical formulation comprising a biologically active agent formulated (e.g., in a preparation, such as a laboratory-grade or pharmaceutical composition) with tryptophan alone or in combination with one or more additional aromatic amino acids and methionine. A preferred combination of amino acids is tryptophan and methionine which together effectively protect against all of the most common mechanisms of protein oxidation.

In another aspect of the invention, compositions of the present invention may also comprise free nucleotides or analogs thereof. Nucleic acid derivatives can be added to parenteral formulations of proteins and peptides, singularly or in combination with methionine. Formulations wherein one or more free nucleotides are present as stabilizers typically contain an amount ranging from about 0.1 to 10 mg/mL. In a particular embodiment, one or more free nucleotides are combined with one or more free aromatic amino acids. A preferred embodiment would comprise free nucleotides combined with methionine.

In another aspect of the invention, compositions of the present invention may also comprise vitamin derivatives such as trolox (6-hydroxy-2,5,7,8-tetramethylchroman-2-carboxylic acid; a water soluble vitamin derivative) and pyridoxine (commonly known as vitamin B6). In a particular embodiment, one or more vitamin derivatives are combined with one or more free aromatic amino acids. A preferred embodiment would comprise vitamin derivatives combined with methionine.

Compositions of the present invention can further contain one or more agents which neutralize free radicals of oxygen (i.e., an ROS scavenger). Suitable ROS scavengers include, for example, mannitol, methionine and/or histidine. Accordingly, in another embodiment, the invention provides a composition containing one or more proteins formulated together with an aromatic amino acid, and one or more ROS scavengers, such as mannitol, methionine and/or histidine. Metal chelating agents, such as EDTA, may also be used as it may inhibit the start of ROS generation.

Compositions of the present invention can also include one or more agents which inhibit protein aggregation. In a particular embodiment, the agent is selected from TWEEN, polysorbate 80, polysorbate 20, glycerol and poloxamer polymers. The compositions can still further include a buffer that maintains the pH of the composition preferably from about 5.0 to about 8.0. Suitable buffers include, for example, histidine, Tris, acetate, MES, succinic acid, PIPES, Bis-Tris, MOPS, ACES, BES, TES, HEPES, EPPS, ethylenediamine, phosphoric acid, and maleic acid. Compositions may also contain tonicifiers such as sodium chloride, arginine salts, etc.

"Surfactants" are molecules with well defined polar and non-polar regions that allow them to aggregate in solution to form micelles. Depending on the nature of the polar area, surfactants can be non-ionic, anionic, cationic, and Zwitterionic. Most parentally acceptable nonionic surfactants come from either the polysorbate or polyether groups. Polysorbate 20 and 80 are contemporary surfactant stabilizers in marketed protein formulations.

Peroxides are known contaminants of non-ionic surfactants. Peroxides in polysorbates can result in oxidative degradation of proteins. Formulators tend to screen sources of polysorbates and other polymeric additives in protein formulations for peroxide contamination and establish peroxide specifications for using the additive. Alternatively, incorporation of an antioxidant is used to help to overcome the potential for non-ionic surfactants to serve as oxidative catalysts for oxygen-sensitive proteins.

Any suitable protein or polypeptide of interest which is susceptible to oxidation can be protected and, thus, stabilized according to the present invention (i.e., can be formulated in an oxidation protected composition as described herein). The protein can be in its natural (e.g., native) form state or be modified by, for example, microencapsulation or conjugation. The protein can be therapeutic or diagnostic. Such proteins include, for example, immunoglobulins, peptides, proteins, and analogs thereof against oxidative damage.

In addition, multi-subunit proteins, such as antibodies, which are particularly susceptible to oxidative damage, protein aggregation and breakdown, rendering them diagnostically and therapeutically non-functional, can be protected according to the present invention. In a particular embodiment, the invention provides protected (i.e., stabilized) antibody compositions, such as those which include one or more monoclonal antibodies, including fully human antibodies, as well as fragments thereof and immunoconjugates (i.e., antibodies conjugated to therapeutic agents, e.g., as a toxin, a polymer, an imaging agent or a drug).

While the preferred embodiments of the present invention relate to compositions and methods for protecting proteins against damage due to oxidation, the biologically active agent can also be selected from the group consisting of peptides, small molecules, carbohydrates, nucleic acids, lipids, proteins, antibodies and/or analogs thereof.

Herein, numerical ranges or amounts prefaced by the term "about" expressly include the exact range or exact numerical amount.

The term "pharmaceutical formulation" refers to a preparation which is in such form as to permit the biological activity of the active ingredient to be effective, and which contains no additional components which are unacceptably toxic to a subject to which the formulation would be administered.

An antibody possesses "biological activity" in a pharmaceutical formulation, if the biological activity of the antibody at a given time is within about 10% (within the errors of the assay) of the biological activity exhibited at the time the pharmaceutical formulation was prepared, as determined by the ability of the antibody *in vitro* or *in vivo* to bind to antigen and result in a measurable biological response.

A "stable" formulation is one in which the protein therein essentially retains its physical and/or chemical stability upon storage. Stability can be measured at a selected temperature for a selected time period. Preferably, the formulation is stable at room temperature (∼30°C) or at 40°C for at least 1 month and/or stable at about 2-8°C for at least 1 year and preferably for at least 2 years. For example, the extent of aggregation during storage can be used as an indicator of protein stability. Thus, a "stable" formulation may be one wherein less than about 10% and preferably less than about 5% of the protein is present as an aggregate in the formulation. Various analytical techniques for measuring protein stability are available in the art and are reviewed, for example, in Peptide and Protein Drug Delivery, 247-301, Vincent Lee Ed., Marcel Dekker, Inc., New York, N.Y., Pubs. (1991) and Jones, A. Adv. Drug Delivery Rev. 10: 29-90 (1993).

The term "aqueous solution" refers to a solution in which water is the dissolving medium or solvent. When a substance dissolves in a liquid, the mixture is termed a solution. The dissolved substance is the solute, and the liquid that does the dissolving (in this case water) is the solvent.

By "inhibiting" oxidation it is intended as preventing, reducing, or decreasing the amount of oxidation, measured by comparing the amount of oxidation present in a protein-containing solution that comprises at least one inhibitor of oxidation with the amount of oxidation present in a protein-containing solution that does not comprise at least one inhibitor of oxidation.

An "oxidized" protein or antibody herein is one in which one or more amino acid residue(s) thereof has been oxidized.

A protein or antibody that is "susceptible to oxidation" is one comprising one or more residue(s) that has been found to be prone to oxidation.

Methods which may find use in the present invention for measuring oxidation of proteins include gel electrophoresis, isoelectric focusing, capillary electrophoresis, chromatography such as size exclusion chromatography, ion-exchange chromatography, and reversed-phase high performance liquid chromatography, peptide mapping, oligosaccharide mapping, mass spectrometry, ultraviolet absorbance spectroscopy, fluorescence spectroscopy, circular dichroism spectroscopy, isothermal titration calorimetry, differential scanning calorimetry, analytical ultra-centrifugation, dynamic light scattering, proteolysis, and crosslinking, turbidity measurement, filter retardation assays, immunological assays, fluorescent dye binding assays, protein-staining assays, microscopy, and other binding assays.

By "polypeptide" or "protein" is meant a sequence of amino acids for which the chain length is sufficient to produce the higher levels of tertiary and/or quaternary structure. Thus, proteins are distinguished from "peptides" which are also amino acid-based molecules that do not have such structure.

The protein which is formulated is preferably essentially pure and desirably essentially homogeneous (*i.e.*, free from contaminating proteins). "Essentially pure" protein means a composition comprising at least about 90% by weight of the protein, based on total weight of the composition, preferably at least about 95% by weight. "Essentially homogeneous" protein means a composition comprising at least about 99% by weight of protein, based on total weight of the composition.

In certain embodiments, the protein is an antibody. The antibody herein is directed against an "antigen" of interest. Preferably, the antigen is a biologically important protein and administration of the antibody to a mammal suffering from a disease or disorder can result in a therapeutic benefit in that mammal. However, antibodies directed against non-protein antigens (such as tumor-associated glycolipid antigens; see US Patent 5,091,178) are also contemplated. Where the antigen is a protein, it may be a transmembrane molecule (*e.g*., receptor) or ligand such as a growth factor. Exemplary antigens include those proteins discussed above. Preferred molecular targets for antibodies encompassed by the present invention include CD polypeptides such as CD3, CD4, CD8, CD 19, CD20, CD34 and CD40; members of the HER receptor family such as the EGF receptor (HER1), HER2, HER3 or HER4 receptor; cell adhesion molecules such as LFA-1, Mac1, p150,95, VLA-4, ICAM-1, VCAM and av/b3 integrin including either α or β subunits thereof (*e.g*., anti-CD11a, anti-CD18 or anti-CD11b antibodies); macrophage receptor such as CRIg, tumor necrosis factors such as TRAIL/Apo-2, growth factors such as vascular endothelial growth factor (VEGF); IgE; blood group antigens; flk2/flt3 receptor; obesity (OB) receptor; mpl receptor; CTLA-4; polypeptide C etc. Other exemplary proteins include growth hormone (GH), including human growth hormone (hGH) and bovine growth hormone (bGH); growth hormone releasing factor; parathyroid hormone; thyroid stimulating hormone; lipoproteins; α-1 -antitrypsin; insulin A-chain; insulin B-chain; proinsulin; follicle stimulating hormone; calcitonin; luteinizing hormone; glucagon; clotting factors such as factor VIIIC, factor, tissue factor, and von Willebrands factor; anti-clotting factors such as Protein C; atrial natriuretic factor; lung surfactant; a plasminogen activator, such as urokinase or tissue-type plasminogen activator (t-PA); bombazine; thrombin; tumor necrosis factor-α and -β; enkephalinase; RANTES (regulated on activation normally T-cell expressed and secreted); human macrophage inflammatory protein (MIP-1-α); serum albumin such as human serum albumin (HSA); mullerian-inhibiting substance; relaxin A-chain; relaxin B-chain; prorelaxin; mouse gonadotropin-associated peptide; DNase; inhibin; activin; receptors for hormones or growth factors; an integrin; protein A or D; rheumatoid factors; a neurotrophic factor such as bone-derived neurotrophic factor (BDNF), neurotrophin-3, -4, -5, or -6 (NT-3, NT-4, NT-5, or NT-6), or a nerve growth factor such as NGF-β; platelet-derived growth factor (PDGF); fibroblast growth factor such as aFGF and bFGF; epidermal growth factor (EGF); transforming growth factor (TQF) such as TGF-α and TGF-β, including TGF-β1, TGF-β2, TGF-β3, TGF-β4, or TGF-β5; insulin-like growth factor-I and -II (IGF-I and IGF-II); des(1-3)-IGF-I (brain IGF-I); insulin-like growth factor binding proteins (IGFBPs); erythropoietin (EPO); thrombopoietin (TPO); osteoinductive factors; immunotoxins; a bone morphogenetic protein (BMP); an interferon such as interferon-α, -β, and -γ; colony stimulating factors (CSFs), e.g., M-CSF, GM-CSF, and G-CSF; interleukins (ILs), e.g., IL-1 to IL-10; superoxide dismutase; T-cell receptors; surface membrane proteins; decay accelerating factor (DAF); a viral antigen such as, for example, a portion of the AIDS envelope; transport proteins; homing receptors; addressins; regulatory proteins; immunoadhesins; antibodies; and biologically active fragments or variants of any of the above-listed polypeptides. Many other antibodies and/or other proteins may be used in accordance with the instant invention, and the above lists are not meant to be limiting.

Soluble antigens or fragments thereof, optionally conjugated to other molecules, can be used as immunogens for generating antibodies. For transmembrane molecules, such as receptors, fragments of these (*e.g*., the extracellular domain of a receptor) can be used as the immunogen. Alternatively, cells expressing the transmembrane molecule can be used as the immunogen. Such cells can be derived from a natural source (*e.g*., cancer cell lines) or may be cells which have been transformed by recombinant techniques to express the transmembrane molecule.

Examples of antibodies to be formulated herein include, but are not limited to: HER2 antibodies including trastuzumab (HERCEPTIN®) (Carter et al., Proc. Natl. Acad. Sci. USA, 89:4285-4289 (1992), U.S. Patent No. 5,725,856) and pertuzumab (OMNITARG™) (WO01/00245); CD20 antibodies (see below); IL-8 antibodies (St John et al., Chest, 103:932 (1993), and International Publication No. WO 95/23865); VEGF or VEGF receptor antibodies including humanized and/or affinity matured VEGF antibodies such as the humanized VEGF antibody huA4.6.1 bevacizumab (AVASTIN®) and ranibizumab (LUCENTIS®) (Kim et al., Growth Factors, 7:53-64 (1992), International Publication No. WO 96/30046, and WO 98/45331, published October 15, 1998); PSCA antibodies (WO01/40309); CD11a antibodies including efalizumab (RAPTIVA®) (US Patent No. 6,037,454, US Patent No. 5,622,700, WO 98/23761, Stoppa et al., Transplant Intl. 4:3-7 (1991), and Hourmant et al., Transplantation 58:377-380 (1994)); antibodies that bind IgE including omalizumab (XOLAIR®) (Presta et al., J. Immunol. 151:2623-2632 (1993), and International Publication No. WO 95/19181;US Patent No. 5,714,338, issued February 3, 1998 or US Patent No. 5,091,313, issued February 25, 1992, WO 93/04173 published March 4, 1993, or International Application No. PCT/US98/13410 filed June 30, 1998, US Patent No. 5,714,338); CD18 antibodies (US Patent No. 5,622,700, issued April 22, 1997, or as in WO 97/26912, published July 31, 1997); Apo-2 receptor antibody antibodies (WO 98/51793 published November 19, 1998); Tissue Factor (TF) antibodies (European Patent No. 0 420 937 B 1 granted November 9, 1994); α₄-α₇ integrin antibodies (WO 98/06248 published February 19, 1998); EGFR antibodies (*e.g*., chimerized or humanized 225 antibody, cetuximab, ERBUTIX^{®} as in WO 96/40210 published December 19, 1996); CD3 antibodies such as OKT3 (US Patent No. 4,515,893 issued May 7, 1985); CD25 or Tac antibodies such as CHI-621 (SIMULECT®) and ZENAPAX® (See US Patent No. 5,693,762 issued December 2, 1997); CD4 antibodies such as the cM-7412 antibody (Choy et al., Arthritis Rheum 39(1):52-56 (1996)); CD52 antibodies such as CAMPATH-1H (ILEX/Berlex) (Riechmann et al., Nature 332:323-337 (1988)); Fc receptor antibodies such as the M22 antibody directed against Fc(RI as in Graziano et al., J. Immunol. 155(10):4996-5002 (1995)); carcinoembryonic antigen (CEA) antibodies such as hMN-14 (Sharkey et al., Cancer Res. 55(23Suppl): 5935s-5945s (1995)); antibodies directed against breast epithelial cells including huBrE-3, hu-Mc 3 and CHL6 (Ceriani et al., Cancer Res. 55(23): 5852s-5856s (1995); and Richman et al., Cancer Res. 55(23 Supp): 5916s-5920s (1995)); antibodies that bind to colon carcinoma cells such as C242 (Litton et al., Eur J. Immunol. 26(1):1-9 (1996)); CD38 antibodies, *e.g*., AT 13/5 (Ellis et al., J. Immunol. 155(2):925-937 (1995)); CD33 antibodies such as Hu M195 (Jurcic et al., Cancer Res 55(23 Suppl):5908s-5910s (1995)) and CMA-676 or CDP771; EpCAM antibodies such as 17-1A (PANOREX®); GpIIb/IIIa antibodies such as abciximab or c7E3 Fab (REOPRO®); RSV antibodies such as MEDI-493 (SYNAGIS®); CMV antibodies such as PROTOVIR®; HIV antibodies such as PRO542; hepatitis antibodies such as the Hep B antibody OSTAVIR®; CA125 antibody including anti-MUC16 (WO2007/001851; Yin, BWT and Lloyd, KO, J. Biol. Chem. 276:27371-27375 (2001)) and OvaRex; idiotypic GD3 epitope antibody BEC2; αvβ3 antibody (*e.g*., VITAXIN®; Medimmune); human renal cell carcinoma antibody such as ch-G250; ING-1; anti-human 17-1An antibody (3622W94); anti-human colorectal tumor antibody (A33); anti-human melanoma antibody R24 directed against GD3 ganglioside; anti-human squamous-cell carcinoma (SF-25); human leukocyte antigen (HLA) antibody such as Smart ID10 and the anti-HLA DR antibody Oncolym (Lym-1); CD37 antibody such as TRU 016 (Trubion); IL-21 antibody (Zymogenetics/Novo Nordisk); anti-B cell antibody (Impheron); B cell targeting MAb (Immunogen/Aventis); 1D09C3 (Morphosys/GPC); LymphoRad 131 (HGS); Lym-1 antibody, such as Lym -1Y-90 (USC) or anti-Lym-1 Oncolym (USC/Peregrine); LIF 226 (Enhanced Lifesci.); BAFF antibody (*e.g*., WO 03/33658); BAFF receptor antibody (see *e.g*., WO 02/24909); BR3 antibody; Blys antibody such as belimumab; LYMPHOSTAT -B™; ISF 154 (UCSD/Roche/Tragen); gomilixima (Idec 152; Biogen Idec); IL-6 receptor antibody such as atlizumab (ACTEMRA™; Chugai/Roche); IL-15 antibody such as HuMax-Il-15 (Genmab/Amgen); chemokine receptor antibody, such as a CCR2 antibody (*e.g*., MLN1202; Millieneum); anti-complement antibody, such as C5 antibody (*e.g*., eculizumab, 5G1.1; Alexion); oral formulation of human immunoglobulin (*e.g*., IgPO; Protein Therapeutics); IL-12 antibody such as ABT-874 (CAT/Abbott); Teneliximab (BMS-224818; BMS); CD40 antibodies, including S2C6 and humanized variants thereof (WO00/75348) and TNX 100 (Chiron/Tanox); TNF-α antibodies including cA2 or infliximab (REMICADE®), CDP571, MAK-195, adalimumab (HUMIRA™), pegylated TNF-α antibody fragment such as CDP-870 (Celltech), D2E7 (Knoll), anti-TNF-α polyclonal antibody (*e.g*., PassTNF; Verigen); CD22 antibodies such as LL2 or epratuzumab (LYMPHOCIDE®; Immunomedics), including epratuzumab Y-90 and epratzumab I-131, Abiogen's CD22 antibody (Abiogen, Italy), CMC 544 (Wyeth/Celltech), combotox (UT Soutwestem), BL22 (NIH), and LympoScan Tc99 (Immunomedics), as well as , anti-amyloid beta (Abeta), anti-CD4 (MTRX1011A), anti- EGFL7 (EGF-like-domain 7), anti-IL13, Apomab (anti-DR5-targeted pro-apoptotic receptor agonist (PARA), anti-BR3 (CD268, BLyS receptor 3, BAFF-R, BAFF Receptor), anti-beta 7 integrin subunit, dacetuzumab (Anti-CD40), GA101 (anti-CD20 monoclonal antibody), MetMAb (anti-MET receptor tyrosine kinase), anti-neuropilin-1 (NRP1), ocrelizumab (anti-CD20 antibody), anti-OX40 ligand, anti-oxidized LDL (oxLDL), pertuzumab (HER dimerization inhibitors (HDIs), and. rhuMAb IFN alpha..

Examples of anti-CD20 antibodies include: "C2B8," which is now called "rituximab" ("RITUXAN®") (US Patent No. 5,736,137); the yttrium-[90]-labelled 2B8 murine antibody designated "Y2B8" or "Ibritumomab Tiuxetan" (ZEVALIN®) commercially available from IDEC Pharmaceuticals, Inc. (US Patent No. 5,736,137; 2B8 deposited with ATCC under accession no. HB11388 on June 22, 1993); murine IgG2a "B1," also called "Tositumomab," optionally labelled with ¹³¹I to generate the "131I-B1" or "iodine I131 tositumomab" antibody (BEXXAR™) commercially available from Corixa (see, also, US Patent No. 5,595,721); murine monoclonal antibody "1F5" (Press et al., Blood 69(2):584-591 (1987)) and variants thereof including "framework patched" or humanized 1F5 (WO 2003/002607, Leung, S.; ATCC deposit HB-96450); murine 2H7 and chimeric 2H7 antibody (US Patent No. 5,677,180); humanized 2H7 (WO 2004/056312, Lowman *et al.*,); 2F2 (HuMax-CD20), a fully human, high-affinity antibody targeted at the CD20 molecule in the cell membrane of B-cells (Genmab, Denmark; see, for example, Glennie and van de Winkel, Drug Discovery Today 8: 503-510 (2003) and Cragg et al., Blood 101: 1045-1052 (2003); WO 2004/035607; US2004/0167319); the human monoclonal antibodies set forth in WO 2004/035607 and US2004/0167319 (Teeling et al.,); the antibodies having complex N-glycoside-linked sugar chains bound to the Fc region described in US 2004/0093621 (Shitara et al.,); monoclonal antibodies and antigen-binding fragments binding to CD20 (WO 2005/000901, Tedder et al.,) such as HB20-3, HB20-4, HB20-25, and MB20-11; CD20 binding molecules such as the AME series of antibodies, *e.g*., AME 33 antibodies as set forth in WO 2004/103404 and US2005/0025764 (Watkins et al., Eli Lilly/Applied Molecular Evolution, AME); CD20 binding molecules such as those described in US 2005/0025764 (Watkins *et al.*,); A20 antibody or variants thereof such as chimeric or humanized A20 antibody (cA20, hA20, respectively) or IMMU-106 (US 2003/0219433, Immunomedics); CD20-binding antibodies, including epitope-depleted Leu-16, 1H4, or 2B8, optionally conjugated with IL-2, as in US 2005/0069545A1 and WO 2005/16969 (Carr et al.,); bispecific antibody that binds CD22 and CD20, for example, hLL2xhA20 (WO2005/14618, Chang et al.,); monoclonal antibodies L27, G28-2, 93-1B3, B-C1 or NU-B2 available from the International Leukocyte Typing Workshop (Valentine et al., In: Leukocyte Typing III (McMichael, Ed., p. 440, Oxford University Press (1987)); 1H4 (Haisma et al., Blood 92:184 (1998)); anti-CD20 auristatin E conjugate (Seattle Genetics); anti-CD20-IL2 (EMD/Biovation/City of Hope); anti-CD20 MAb therapy (EpiCyte); anti-CD20 antibody TRU 015 (Trubion).

The term "antibody" as used herein includes monoclonal antibodies (including full length antibodies which have an immunoglobulin Fc region), antibody compositions with polyepitopic specificity, multispecific antibodies (*e.g*., bispecific antibodies, diabodies, and single-chain molecules, as well as antibody fragments (*e.g*., Fab, F(ab')₂, and Fv). The term "immunoglobulin" (Ig) is used interchangeably with "antibody" herein.

The term "monoclonal antibody" as used herein refers to an antibody obtained from a population of substantially homogeneous antibodies, *i.e*., the individual antibodies comprising the population are identical except for possible naturally occurring mutations and/or post-translation modifications (*e.g*., isomerizations, amidations) that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature, 256: 495 (1975), or may be made by recombinant DNA methods (see, *e.g*., U.S. Pat. No. 4,816,567). The "monoclonal antibodies" may also be isolated from phage antibody libraries using the techniques described in Clackson et al., Nature, 352:624-628 (1991) and Marks et al., J. Mol. Biol., 222:581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is (are) identical with or homologous to corresponding sequences in antibodies derived from another species or belonging to another antibody class or subclass, as well as fragments of such antibodies, so long as they exhibit the desired biological activity (U.S. Pat. No. 4,816,567; Morrison et al., Proc. Natl. Acad. Sci. USA, 81:6851-6855 (1984)). Chimeric antibodies of interest herein include "primitized" antibodies comprising variable domain antigen-binding sequences derived from a non-human primate (*e.g*., Old World Monkey, Ape etc.) and human content region sequences.

The term "therapeutic antibody" refers to an antibody that is used in the treatment of disease. A therapeutic antibody may have various mechanisms of action. A therapeutic antibody may bind and neutralize the normal function of a target associated with an antigen. For example, a monoclonal antibody that blocks the activity of the of protein needed for the survival of a cancer cell causes the cell's death. Another therapeutic monoclonal antibody may bind and activate the normal function of a target associated with an antigen. For example, a monoclonal antibody can bind to a protein on a cell and trigger an apoptosis signal. Yet another monoclonal antibody may bind to a target antigen expressed only on diseased tissue; conjugation of a toxic payload (effective agent), such as a chemotherapeutic or radioactive agent, to the monoclonal antibody can create an agent for specific delivery of the toxic payload to the diseased tissue, reducing harm to healthy tissue. A "biologically functional fragment" of a therapeutic antibody will exhibit at least one if not some or all of the biological functions attributed to the intact antibody, the function comprising at least specific binding to the target antigen.

An "intact" antibody is one which comprises an antigen-binding site as well as a CL and at least the heavy chain domains, C_{H}1, C_{H}2 and C_{H}3. The constant domains may be native sequence constant domains (*e.g*., human native sequence constant domains) or amino acid sequence variants thereof. Preferably, the intact antibody has one or more effector functions.

An "antibody fragment" comprises a portion of an intact antibody, preferably the antigen binding and/or the variable region of the intact antibody. Examples of antibody fragments include Fab, Fab', F(ab')₂ and Fv fragments; diabodies; linear antibodies (see U.S. Pat. No. 5,641,870, Example 2; Zapata et al., Protein Eng. 8(10): 1057-1062 [1995]); single-chain antibody molecules and multispecific antibodies formed from antibody fragments.

A "biologically functional fragment" of an antibody comprises only a portion of an intact antibody, wherein the portion retains at least one, and as many as most or all, of the functions normally associated with that portion when present in an intact antibody. In one embodiment, a biologically functional fragment of an antibody comprises an antigen binding site of the intact antibody and thus retains the ability to bind antigen. In another embodiment, a biologically functional fragment of an antibody, for example one that comprises the Fc region, retains at least one of the biological functions normally associated with the Fc region when present in an intact antibody, such as FcRn binding, antibody half life modulation, ADCC function and complement binding. In one embodiment, a biologically functional fragment of an antibody is a monovalent antibody that has an in vivo half life substantially similar to an intact antibody. For example, such a biologically functional fragment of an antibody may comprise an antigen binding arm linked to an Fc sequence capable of conferring in vivo stability to the fragment.

"Humanized" forms of non-human (*e.g*., murine) antibodies are chimeric immunoglobulins, immunoglobulin chains or fragments thereof (such as Fv, Fab, Fab', F(ab')₂ or other antigen-binding subsequences of antibodies) of mostly human sequences, which contain minimal sequence derived from non-human immunoglobulin. For the most part, humanized antibodies are human immunoglobulins (recipient antibody) in which residues from a hypervariable region (also CDR) of the recipient are replaced by residues from a hypervariable region of a non-human species (donor antibody) such as mouse, rat or rabbit having the desired specificity, affinity, and capacity. In some instances, Fv framework region (FR) residues of the human immunoglobulin are replaced by corresponding non-human residues. Furthermore, "humanized antibodies" as used herein may also comprise residues which are found neither in the recipient antibody nor the donor antibody. These modifications are made to further refine and optimize antibody performance. The humanized antibody optimally also will comprise at least a portion of an immunoglobulin constant region (Fc), typically that of a human immunoglobulin. For further details, see Jones et al., Nature, 321:522-525 (1986); Reichmann et al., Nature, 332:323-329 (1988); and Presta, Curr. Op. Struct. Biol., 2:593-596 (1992).

"Isolated" when used to describe the various polypeptides and antibodies disclosed herein, means a polypeptide or antibody that has been identified, separated and/or recovered from a component of its production environment. Preferably, the isolated polypeptide is free of association with all other components from its production environment. Contaminant components of its production environment, such as that resulting from recombinant transfected cells, are materials that would typically interfere with diagnostic or therapeutic uses for the polypeptide, and may include enzymes, hormones, and other proteinaceous or non-proteinaceous solutes. In preferred embodiments, the polypeptide will be purified (1) to a degree sufficient to obtain at least 15 residues of N-terminal or internal amino acid sequence by use of a spinning cup sequenator, or (2) to homogeneity by SDS-PAGE under nonreducing or reducing conditions using Coomassie blue or, preferably, silver stain. Ordinarily, however, an isolated polypeptide or antibody will be prepared by at least one purification step.

"Treatment" refers to both therapeutic treatment and prophylactic or preventative measures. Those in need of treatment include those already with the disorder as well as those in which the disorder is to be prevented.

"Mammal" for purposes of treatment refers to any animal classified as a mammal, including humans, domestic and farm animals, and zoo, sports, or pet animals, such as dogs, horses, rabbits, cattle, pigs, hamsters, gerbils, mice, ferrets, rats, cats, etc. Preferably, the mammal is human.

A "disorder" is any condition that would benefit from treatment with the protein. This includes chronic and acute disorders or diseases including those pathological conditions which predispose the mammal to the disorder in question. Non-limiting examples of disorders to be treated herein include carcinomas and inflammations.

A "therapeutically effective amount" is at least the minimum concentration required to effect a measurable improvement or prevention of a particular disorder. Therapeutically effective amounts of known proteins are well known in the art, while the effective amounts of proteins hereinafter discovered may be determined by standard techniques which are well within the skill of a skilled artisan, such as an ordinary physician.

### II. Modes for Carrying out the Invention

Recent oxidation events on monoclonal antibody candidates prompted us to investigate the mechanism of oxidation on Met, Trp and His residues, and to search suitable stabilizers. By using a model protein, parathyroid hormone (PTH, 1-34), aided by rp-HPLC, peptide mapping and LC/MS/MS analysis, we were able to identify and quantify the oxidation on these vulnerable residues caused by different oxidants.

### A. Characterization of Oxidative Damage

The fact that oxidized methionine (Met[O]) is readily detected in numerous pharmaceutical proteins may be attributed to its susceptibility to oxidizing agents and not just to H2O2 alone. Light, tBHP, and/or peroxodisulfate have been used by various laboratories to generate Met[O]. The oxidation of Trp or His in pharmaceutical proteins under normal storage conditions can be very slow. To expedite oxidation, one or more stress models are commonly used.

Many protein formulations contain polysorbate (20 and 80). It has been reported that the oxidants present in aged polysorbate consist predominantly of H₂O₂ (up to 75%) (Jaeger et al., Biochem Biophys Methods 29:77-81, (1994)). Ha et al. (J Pharm Sci 91:2252-2264 (2002)) reported increased oxidation of an interleukin-2 mutant by aged polysorbate. Since polysorbate is the source of oxidant in protein drug product, one may consider using H₂O₂ as a way to simulate the oxidative reaction in surfactant containing formulations. In addition, H₂O₂ has been used as an aseptic agent for isolator used in filling sterile products. Residual H₂O₂ can be the found in the drug product. For this reason, it is important to determine the sensitivity of the protein to oxidation by H₂O₂.

Trp and His oxidation are considered as metal-catalyzed or free radical-mediated oxidation. (Davies et al. 1987, Hawkins and Davies 2001) Theoretically, metal-catalyzed oxidation would serve as a useful model. In experimental design, however, the selection of metal (e.g. iron or copper) and determining whether or not to add chelating agent (e.g., EDTA) have profound impacts on the outcome of the experimental results. The following examples illustrate the complexity of results achieved from oxidation involving metal. With addition of metal in the oxidizing system, such as ascorbate/Cu(II)/O2, two Met and one His residues in relaxin were oxidized, but none of the two tryptophan residues. With bovine serum albumin, free radicals generated from Fe(II)/EDTA/ascorbate system preferred tryptophan, whereas, Cu(II)/ascorbate (*EDTA excluded*) preferred histidine (Uchida K, Agric Biol Chem 53:3285-92, 1989). H₂O₂/Fe(II)/EDTA and H₂O₂/Cu(II) generated different pattern of albumin degradation (Kocha et al., Biochim Biophys Acta 1337:319-26 (1997)). Via metal catalyzed oxidation, tryptophan and methionine residues in α-crystallin were oxidized by H₂O₂/Fe(II)/EDTA (Finley et al., Protein Sci. 7:2391-7, (1998)).

During pharmaceutical production, recombinant proteins are necessarily exposed to stainless steel; thus, protein solutions may contain trace amounts of iron or other metals. Therefore, we chose H2O2 with Fe(II) - the commonly known Fenton reaction - as a stress condition to evaluate the oxidation potential of our drug candidates.

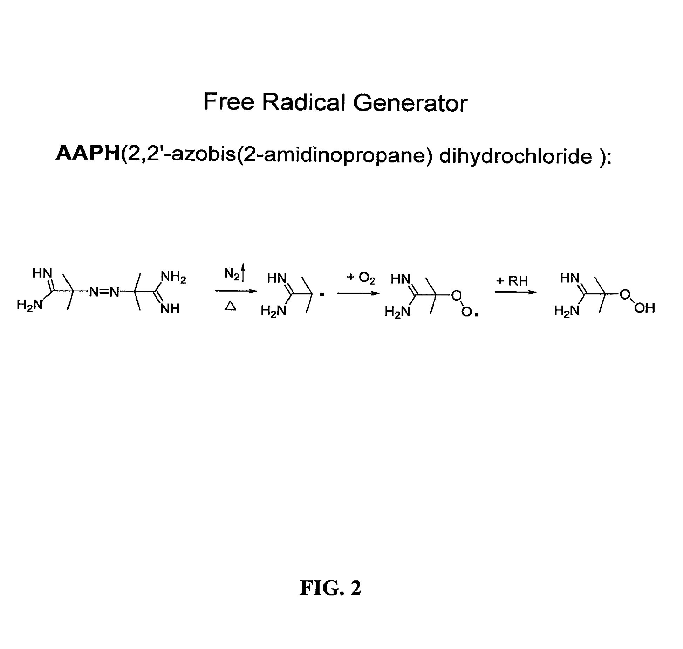

As discussed in previous paragraph, the presence of metal increases the complexity of oxidation studies. AAPH (2,2'-azobis(2-amidinopropane) dihydrochloride) is a metal-ion independent, reactive-oxygen-species (ROS) generating system (Niki et al. Methods Enzymology. 186: 100-8, 1990). At a defined rate, it decomposes in aqueous, aerobic solutions to yield alkyl radicals and alkylperoxides. The chemical structure and generation of alkylperoxides are shown in Figure 2. Treatment with AAPH led to oxidation of Met, Tyr and Trp residues in liver proteins (Chao et al., Proc Natl Acad Sci 94:2969-74, (1997)). In the same study, another amino acid derivative from oxidation, dityrosine was also detected. When glutamine synthetase was exposed to AAPH for 4 hour, both Trp residues, 2 of 16 His, 6 of 17 Tyr and 5 of 16 Met were lost (Ma et al., Arch Biochem Biophys 363:129-134, (1999)). These two reports indicated that AAPH led to the oxidation of a wide range of amino acids in addition to Met. More recently, with respect to small molecule drugs, AIBN (2,2'-azobisisobutyronitrile) and ACVA (4,4'-azo-bis-4-cyanovaleric acid, both azo compounds similar to AAPH, were evaluated for use in the oxidative forced degradation studies (Nelson ED, J Pharm Sci 95:1527-39, (2006)). Because azo compounds can generate reproducible amount of radicals, independently of metals, AAPH is a model oxidant for its ability specifically to generate Trp-oxidized protein.

For non-site-specific oxidation, parathyroid hormone (1-34) (PTH) was chosen as a model protein because of its minimal tertiary structure (Barden et al., J Biochem, 32:7126-32 (1993)) and its sequence containing all three desirable amino acids (1 Trp, 2 Met and 3 His), the ease with which it can be assayed by reversed-phase high-performance liquid chromatography (rp-HPLC), and its availability. Trout's group at the Massachusetts Institute of Technology studied Met oxidation in PTH stressed only by H₂O₂, the different oxidation rates of Met8 and Met18 were found to correlate to 2-shell water coordination number (Chu et al., Biochem 43: 14139-48 (2004) and J Pharm Sci 93:3096-102 (2004)). The difference, less than 1.5 fold, was not sufficiently significant to influence the conclusion that we would draw from our study. The oxidation rates of different Met residues in growth hormone (The et al., J Biol Chem 262:6472-7, (1987)) and rhVEGF (Duenas et al., Pharm Res 18:1455-60, (2001)) were attributed primarily to different degrees of solvent exposure. We would expect the oxidation rate of the fully solvent-exposed Met in PTH, growth hormone and rhVEGF to be comparable. Therefore, the two Met residues on PTH can simulate solvent exposed Met in all proteins. Ease of analysis by LC/MS because of only one Trp, makes PTH a good model protein for our study.

While H₂O₂, tBHP with and without Fe(II) oxidized primarily the two Met residues, AAPH and H₂O₂ plus Fe(II) oxidized Met and Trp residues, with the former more capable to generate Trp[O] species than the latter. AAPH, a metal-independent free radical generator, produced alkylperoxides, which simulated the reactive oxidizing species generated from degraded Tween.

On the otherhand, site-specific metal-catalyzed oxidation will be quite different from a reaction generated only with H2O2. For example, in relaxin, Met-B(25) reacted with H₂O₂ faster than did Met-B(4). The order was reversed when relaxin reacted with Asc/Cu(II) by way of a metal catalyzed reaction, Met-B(4) which is near the metal binding site, tends to be oxidized faster (Li et al., Biochem 34:5762-72 (1995)). In our study, we used a humanized anti-VEGF antibody fragment, indicated for the treatment of neovascular (wet) age-related macular degeneration, as the protein susceptible to site-specific metal-catalyzed oxidation because we have found that Trp 50(H2) oxidation can be inhibited by the addition of EDTA, moreover, when neighboring His was mutated out, Trp 50(H2) became stable (manuscript in preparation). We also studied the oxidation of An anti-CD11a antibody designed to selectively and reversibly block the activation, reactivation and trafficking of T-cells that lead to the development of psoriasis, because it has been oxidized in various conditions, in which different Met[O] products have been observed. However, no Trp oxidation was found in anti-CD11a antibodies under these stressed conditions, a very different behavior than anti-VEGF antibody. For this reason, The anti-CD11a antibody was included in our study.

### B. Screening Stabilizers for the Prevention of Oxidative Degradation

A key objective in this study was to screen stabilizers. It is anticipated that the information generated in these stress studies might lead us to a novel stabilizer for use in pharmaceutically effective preparations. It is prudent to screen stabilizers by using H₂O₂, H₂O₂ plus Fe(II) and AAPH, as they represent potential assaults from vapor H₂O₂ commonly used as aseptic agent, H₂O₂ from degraded Tween and metal from stainless steel surface and alkylperoxides also from degraded Tween, respectively. From our screening study we determined that 1) free methionine protected PTH oxidation against H₂O₂ and H₂O₂ plus Fe(II), 2) mannitol and EDTA were effective against H₂O₂ plus Fe(II), 3) free tryptophan was effective only against the oxidation by AAPH, whereas the combination of Trp and Met was effective against all three oxidant conditions.

The anti-VEGF antibody oxidation represents site-specific metal-catalyzed oxidation. AAPH could generate Anti-VEGF antibody degradants which showed extra peaks in basic region in IEC chromatograms as the trouble qualification lot. Free Trp was effective in mitigating oxidation in Anti-VEGF antibodies when oxidized by AAPH. These results suggest that free Trp is effective against site-specific metal-catalyzed oxidation. In order to assure all vulnerable amino acid residues such as methionine, tryptophan, and possibly histidine are protected, a combination of methionine and tryptophan combination will be an effective measure.

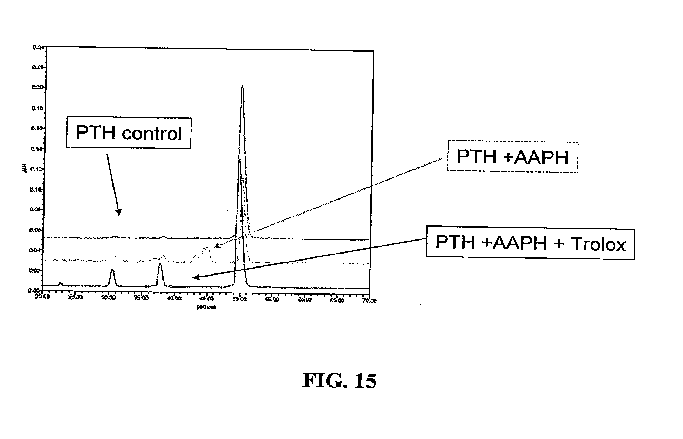

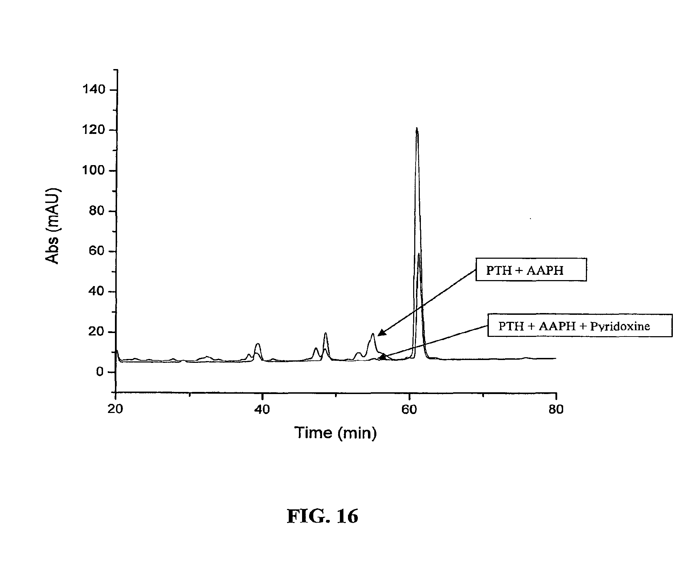

Other than tryptophan being a good stabilizer in combination with methionine, we have also demonstrated that trolox (6-hydroxy-2,5,7,8-tetramethylchroman-2-carboxylic acid; a water soluble vitamin derivative) and pyridoxine (commonly known as vitamin B6) both showed excellent protection of tryptophan residues in a protein (Figures 15 and 16). Each of these stabilizers was separately tested at a 2 mg/mL concentration by addition to the 0.1 mg/mL protein solution of PTH, pH 5.0. The protein solution was subsequently stressed by the addition of 1 mM of AAPH, and incubated at 40°C for 6 hours. Without the protection of trolox or pyridoxine, PTH exhibited significant amount of oxidation at its tryptophan residue, whereas in the presence of trolox or pyridoxine, the tryptophan residue was well protected (Figures 15 and 16). These results affirmed the utility of using a free radical scavenger to protect tryptophan residues in a protein.

### EXAMPLE 1

### A Study of Protein Oxidation: Methionine and Tryptophan as Effective Stabilizers Against Oxidative Degradation Mechanisms

This example illustrates the use of tryptophan alone and in combination with methionine to prevent oxidation of antibodies and proteins.

### EXPERIMENTAL METHODS

### Material:

AAPH (2,2'-azobis(2-amidinopropane) dihydrochloride) (lot# D00024287) was purchased from CalBiochem (Gibbstown, NJ). Parathyroid hormone (1-34) (SVSEIQLMHNLGKHLNSMERVEWLRKKLQDVHNF, lot # U07046A1) was purchased from American Peptide Company (Sunnyvale, CA). In this report, it is simply referred as PTH.

L- Methionine and EDTA disodium (lot# E05643) were purchased from J.T. Baker (Phillipsburg, NJ). Sodium acetate, ammonium acetate, H₂O₂, t-BHP, L-Tryptophan (lot# 1152333), and ferric chloride hexahydrate (lot # 53H0619) were purchased from Sigma-Aldrich (St. Louis, MO). Ferrous chloride tetrahydrate (lot # NA1759) was purchased from EMD (Gibbstown, NJ). Mannitol (G10303, lot# 139476) and sucrose (G20244, lot# 292426) were obtained within Genentech, Inc. Trypsin, sequencing grade (TPCK treated), was purchased from Promega (Madison, WI). HPLC grade acetonitrile (ACN) and water were purchased from Fisher Scientific (Fairlawn, NJ). Water used in sample preparation experiments was obtained from a Milli-Q Plus purification system (Millipore, Bedford, MA).

### Sample preparation

PTH (0.1 mg/mL) was mixed with H₂O₂, H₂O₂/Fe(II), t-BHP, t-BHP/Fe(II), or AAPH, respectively at a molar ratio of 1:42 (protein: oxidant) in 20mM ammonium acetate buffer at pH 5.0. The concentration of Fe(II) was 0.2mM. Details of the compositions are presented in Table 1. As shown in the parenthesis the final concentration in the test samples, mannitol (15%), sucrose (6%), Met (2mg/mL), EDTA (0.04%), and Trp (2mg/mL) were added to these samples as stabilizers at respective concentrations. For anti-VEGF antibody samples, Trp concentrations at 2 and 10 mg/mL were tested. After incubation at 40°C for 6 and 24 hours aliquots of samples were mixed with methanol and Met to quench the reaction prior to rp-HPLC analysis, peptide mapping, and liquid chromatography/mass spectrometry/mass spectrometry (LC/MS/MS). Reconstituted anti-CD11a antibody lyophilized formulation, in liquid form, was tested at 92 mg/mL with 31 mM of AAPH. In a denatured condition, 5 mg/mL anti-CD11a antibody was denatured using 6M guanidine HCl and 1.7 mM AAPH was added.

### Reversed-phase chromatography (rp-HPLC)

The experiments were carried on a Waters HPLC instrument using a C4 (Vydac, 214TP, 5µ, 2.1 x 250 mm). The solvent A was 0.1% trifluoracetic acid (TFA) in H₂O and the solvent B was 0.08% TFA in acetonitrile. The samples were analyzed with a linear gradient from 20% B to 80% B at a flow rate of 0.2 mL/min in 45 min. The column temperature was set at 30°C. The UV detection was set at 214 nm.

### Trypsin digestion

The pH of the samples was adjusted to >7.5 by adding 1 M ammonium bicarbonate. Five microliters of 0.5 mg/mL trypsin was added to 200 µL samples, which were then incubated at 37°C for 3 to 4 hours. The digestion was quenched with 0.1% TFA.

### LC/MS/MS characterization of the tryptic peptide map:

PTH samples after tryptic digestion were separated with an Agilent 1200 Series HPLC system, and the masses and sequences of the peptides were determined with an online-coupled LTQ linear ion-trap mass spectrometer (Thermo Electron, San Jose, CA). A Jupiter Proteo 1.0 x 150 mm column (particle size 4 µm, pore size 90 Å; Phenomenex, Torrance, CA) was used; its temperature was controlled at 30°C, and the column effluent was monitored at 214 nm. The flow rate was controlled at 150 µL/min, and the mobile phases used were 0.1 % TFA in water (A) and 0.1 % TFA in acetonitrile (B). A 100 µL volume of the sample was injected. The optimized gradients (expressed as minutes per %B) were 0/2%, 3/2%, 10/8%, 15/8%, 60/40%, 61/95%, 65/95%, 66/2%, and 76/2%. The effluent from the HPLC was directly infused into the LTQ electrospray ionization source. Electrospray ionization in positive ion mode was achieved by using a needle spray voltage of 4.5 kV and a capillary voltage of 44 V. In the LC/MS/MS experiments, nine scan events, including a full scan in the range of 300 to 2000 m/z, were followed by four cycles of zoom scans and MS/MS scans on the four most intense ions.

MS/MS spectra interpretation and peptide assignments were accomplished with an automatic database search with a SEQUEST algorithm using BioWorks Browser version 3.2 software (Thermo Electron) and manual investigation of each matched product ion spectrum. A FASTA single-protein database of PTH was created and used as the searching target. For the identification of oxidation products, oxidation-related modifications were defined as variable ones (+4, +16, and +32 Da for Trp; +16 Da for Met; and +16 , -22, and -23 Da for His, relative to PTH). Peptide matches with satisfied correlation-factor values (Xc≥1.5 for singly charged, ≥2.0 for doubly charged, and ≥2.5 for triply charged peptide ions) were selected as potentially significant matches for an oxidation-modified peptide. Subsequently, manual investigation of zoom-scan mass spectra and MS/MS spectra of the matched peptide ions was performed to eliminate false positive identifications. Zoom-scan MS profiles were examined to confirm the charge state and monoisotopic mass of matched peptides. To estimate the oxidation level for each oxidation site, the extracted ion chromatograms of corresponding peptides were manually integrated using an Xcalibur Qual Browser. The relative percentage of oxidation was subsequently calculated by dividing the peak area of the oxidized peptide ion by the sum of the peak areas of oxidized and non-oxidized peptides.

### RESULTS AND DISCUSSION

### Non-site-specific Oxidation, PTH:

PTH containing no metal binding site, is a model protein to study non-site-specific oxidation. Reaction takes place on the solvent exposed residues. PTH, at 0.1 mg/mL was allowed to react at 40°C with oxidant, all at 1mM, for 6 and 24 hours. The oxidant to PTH molar ratio was 41.2. Total of five oxidants were used, namely, AAPH, H₂O₂ and tBHP with or without Fe (II). The reactants are summarized in Table 1. Samples were analyzed by rp-HPLC and tryptic peptide mapping followed by LC/MS/MS characterization.

**Table 1**

| PTH | H₂O₂ | Fe²⁺ | t-BHP | AAPH | Ratio [O]/PTH |
|---|---|---|---|---|---|
| 0.1 mg/ml control | | | | | |
| 0.1 mg/ml | 34ppm (1mM) | | | | 41.2 |
| 0.1 mg/ml | 34ppm | 50ppm (1mM) | | | 41.2 |
| 0.1 mg/ml | | | 90ppm (1mM) | | 41.2 |
| 0.1 mg/ml | | 50ppm | 90ppm | | 41.2 |
| 0.1 mg/ml | | | | 1mM | 41.2 |

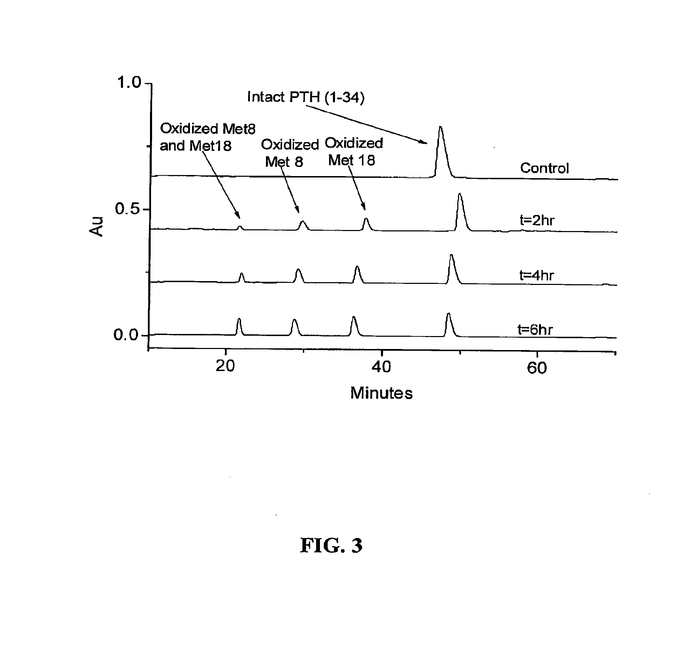

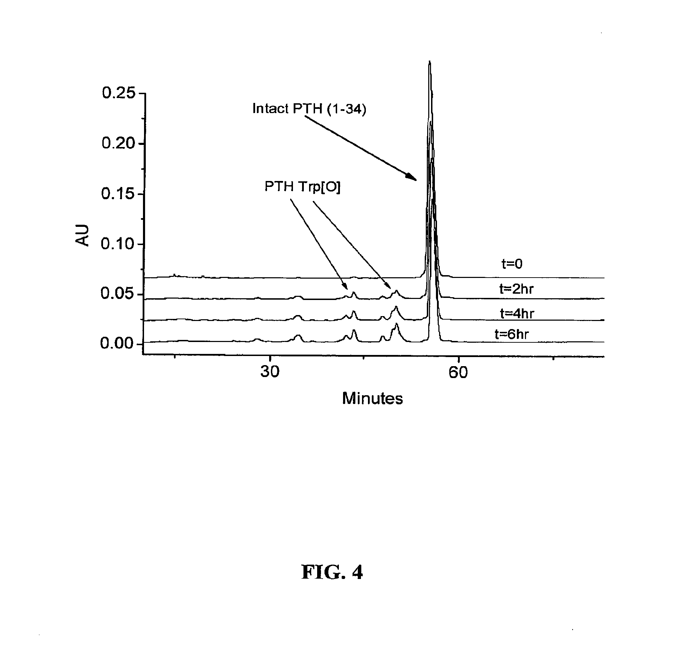

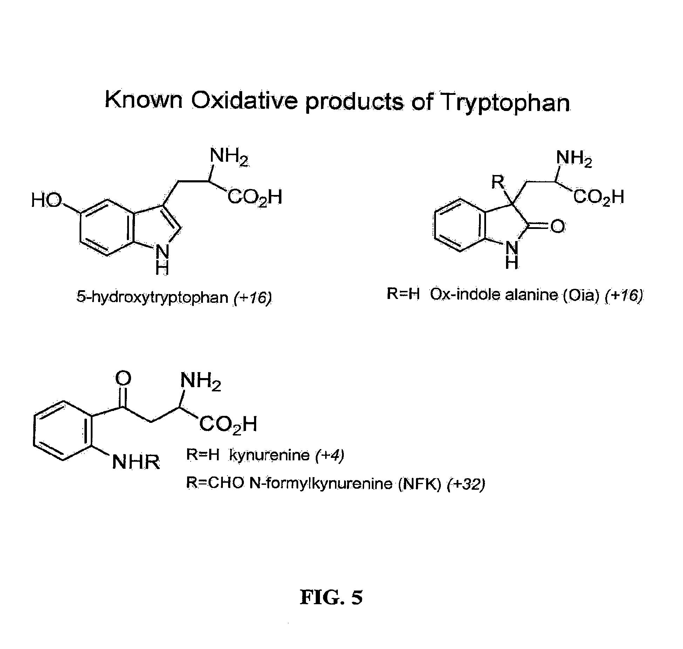

Fig. 3 shows the rp-HPLC chromatograms of PTH reacted with H₂O₂, in which Met18-modified, Met8-modified, and doubly modified PTH species were detected. This trend is consistent with data generated by Chu et al., Biochem 43:14139-48 (2004)) who reported the three Met- oxidized species as detected by rp-HPLC, with Met18 oxidized more than Met8, followed by the doubly oxidized . Fig. 4 shows the rp-HPLC chromatograms of PTH reacted with AAPH and reveals a very different pattern from that shown in Fig. 3. Two sets of triplet peaks appeared at the retention times between PTH and Met[O] peaks. Although the individual peaks were not fully characterized, it was later confirmed by tryptic digestion, followed by LC/MS/MS, that these new peaks were Trp[O]-modified PTH species. Tryptic peptide mapping of the PTH digests found that, in addition to the Met oxidation products, three tryptic peptide species with molecular masses of M+4, M+32, M+16 (where M is the mass of tryptic peptide VGWLR of PTH) were produced when PTH was treated with AAPH. Analysis of MS/MS spectra of the peptide species resulted in their assignment as three Trp oxidation derivatives, namely, kynurenine (M+4), N-formylkynurenine (M+32) and 5-hydroxytryptophan or ox-indole alanine (M+16). Their chemical structures are shown in Fig. 5. The rationales for testing PTH by three model oxidants (e.g., H2O2, H2O2 plus Fe(II), and AAPH) are discussed above. tBHP and tBHP plus iron were also tested because tBHP is currently the oxidant of choice in protocols for degraded-sample preparation.

Table 2 summarizes the overall oxidation of Met8 and Trp23 of PTH in these degraded samples. Altogether, 43% and 84% of the Trp residues of PTH were oxidized by AAPH when treated for 6 hours and 24 hours, respectively. Hence, the new peaks shown in Figure 2 were identified as Trp[O]-modified PTH species. No His oxidation was observed in the experiment. Oxidized Met18 containing tryptic peptide was not retained on the reverse-phase column. Table 2 summarizes the overall oxidation of Met 8 and Trp 23 of PTH in these degraded samples.

**Table 2**

| **Quantitation of PTH Oxidation (Trp23, Met8) by Peptide Mapping** | | | | | | | | |
|---|---|---|---|---|---|---|---|---|
| Oxidants | | Met 8 | Met + 16 | Trp 23 | Total Trp [O] | Trp + 16 | Trp + 32 | Trp + 4 |
| AAPH | 6hr | 71 | 29 | 57 | 43 | 35.1 | 6.4 | 1.3 |
| | 24 hr | 42 | 58 | 16 | 84 | 61 | 20.3 | 2.9 |
| H₂O₂ | 6 hr | 59 | 41 | 99 | 1 | 0.6 | 0.5 | 0.1 |
| | 24 hr | 17 | 83 | 98 | 2 | 0.8 | 0.5 | 0.2 |
| H₂O₂ + Fe | 6 hr | 44 | 55 | 82 | 18 | 11.3 | 5.3 | 1.1 |
| | 24hr | 9 | 91 | 65 | 35 | 22.1 | 10.8 | 2.2 |
| tBHP | 6 hr | 91 | 9 | 100 | 0 | 0 | 0 | 0 |
| | 24 hr | 82 | 18 | 100 | 0 | 0 | 0 | 0 |
| tBHP + Fe | 6 hr | 90 | 10 | 97 | 3 | 2 | 0.8 | 0.2 |
| | 24hr | 78 | 22 | 97 | 3 | 2.1 | 0.6 | 0.2 |
| Control | 6 hr | 100 | 0 | 100 | 0 | 0 | 0 | 0 |
| | 24hr | 99 | 1 | 100 | 0 | 0 | 0 | 0 |

The key observations from these results are as follows:
a. Three conditions, tBHP with or without iron, and H₂O₂, generated minimal amount of Trp oxidation.
b. None of the three His residues was affected.
c. Only AAPH and the Fenton reaction, H₂O₂ plus Fe(II), generated Trp oxidation.
d. More +16 Trp[O] than other species (+4 and +32) was generated.
e. To reach a comparable degree of Trp oxidation, AAPH treatment for 6 hours generated 43% Trp[O] and 29% of Met[O] at Met8, whereas H₂O₂/Fe(II) treatment for 24 hours generated 35% Trp[O] but a much larger amount (91 %) of Met[O] at Met8. This comparison shows that AAPH treatment is more specific toward Trp oxidation than is the Fenton reaction.

The mechanism of thioether (methionine) oxidation by peroxides (H₂O₂, tBHP, or other ROOH species) is a one-step nucleophilic attack of sulfide on a peroxide-protic solvent complex followed by a series of concerted electronic displacements leads to the transfer of oxygen to the sulfur atom, and resulted in Met sulfoxide, Met[O]. (Li et al. Biotechnol Bioeng. 48: 490-500, 1995) This reaction mechanism implies that the peroxide oxygen is electrophilic. Thus electron-donating group such as t-butyl decelerate the reaction by decreasing the electrophilicity of oxygen. For this reason, the fact that tBHP generated less amount of oxidation in Table 2 is not surprising. It should be pointed out that tBHP offers advantage of oxidizing only the exposed methionine as Keck (Anal Biochem 236:56 (1996)) first reported when recombinant interferon gamma (rIFN-γ ;Actimmune) and recombinant tissue plasminogen activator (rtPA; alteplase, ACTIVASE^{®}) were investigated. Since there is little tertiary structure in PTH, we do not expect any Met in PTH to be selectively oxidized by t-BHP.

Although the mechanism of nucleophilic attack predicts a specific acid catalysis component, the reaction rate does not vary significantly in the range of pH 2-8, as shown with PTH (Chu et al., Proc Natl Acad Sci 94:2969-74 (2004)). For this reason, data generated using pH 5 in acetate buffer can be applicable to a typical pH range, pH 5-7, found in protein formulations.

Only AAPH and Fenton reaction generated Trp oxidation. This result supports the notion that nucleophilic reaction of H₂O₂ alone can not cause Trp to oxidize. We were surprised to observe no His oxidation at all in our experiment. When bovine serum albumin reacted with Fe(II)/EDTA/ascorbate or Cu(II)/ascorbate, the former caused more oxidation on Trp, whereas the latter caused more His oxidation (Uchida et al. Agric Biol Chem. 53: 3285-92 (1989)). In another laboratory, relaxin oxidation by AscA/Cu(II) resulted in a significant amount, and AscA/Fe(III) in small amount, of His oxidation, at the same time, neither Trp nor Tyr oxidation was noted (Li et al., Biochem 34: 5762-72 (1995)). The results from both laboratories, contradict the total absence of His oxidation in PTH when H₂O₂+ Fe(II) was used. It is possible that His oxidation depends on the presence of copper.

### Screening stabilizer, PTH:

Based on the analysis above, we propose that protein may be susceptible to oxidative attack via any or all three degradation mechanisms shown in Figure 1, as well as light-induced oxidation. A nucleophilic reaction with H₂O₂ (and no metal) can be the oxidation reaction observed when the protein product is exposed to the vapor H₂O₂ used as aseptic agent in isolator, or to the H₂O₂ resulting from the degradation of Tween (Jaeger et al., Biophy Method 29:77-81 (1994)) When trace metal (iron, copper, or chromium) is introduced into the formulation solution as a result of contact with stainless steel, the Fenton reaction- H₂O₂ with Fe(II)- is operative. The third mechanism is via alkylperoxides which could come from degraded Tween. (Jaeger et al., Biophy Method 29:77-81 (1994)). In the present study, AAPH was used to simulate the reactive oxygen species resulting from alkylperoxides (Figure 2).

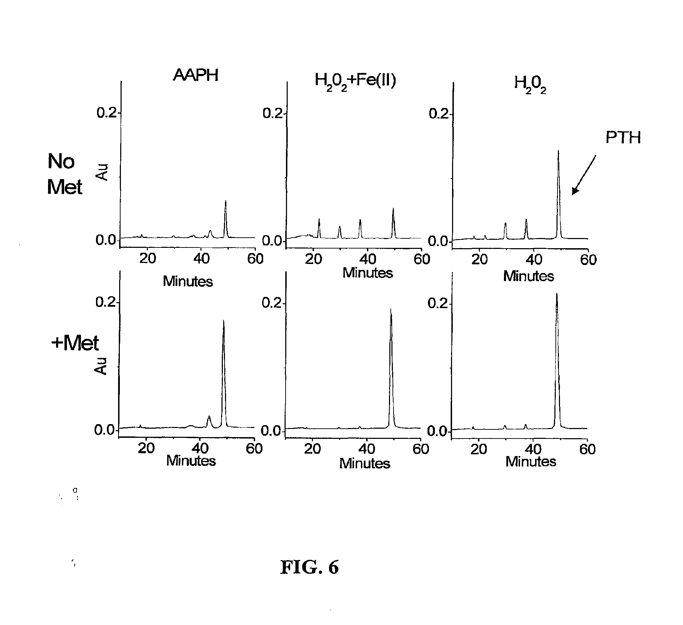

**Methionine:** Free Met neutralized the effect of the oxidants H₂O₂ as expected, whether iron is present or not. Free Met significantly reduced the oxidation of Met residues in PTH, as the peaks corresponding to Met[O]-PTH did not appear (Figure 6). Free Met had no effect on the oxidation of Trp, as Trp[O] peak persisted.

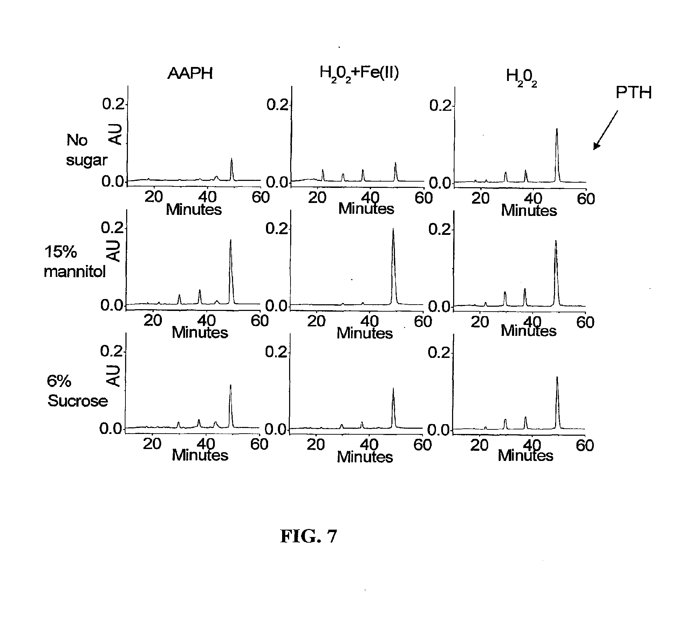

**Mannitol, Sucrose:** Mannitol is a well known hydroxyl free radical scavenger. Figure 7 shows complete protection of Fenton reaction by mannitol, as evidenced by the absence of any Met[O]- and Trp[O]- derived PTH when it was stressed with H2O2/Fe(II). However, when stressed by AAPH or by H₂O₂, PTH was not protected by mannitol at all, because mannitol does not react with alkylperoxides or H₂O₂. Sucrose generated similar results, except it was less effective than mannitol when protecting PTH against a hydroxyl free radical. Polyols were considered a universal stabilizer against both physical and chemical degradations. As noted in a review by Li et al.(Biotech Bioeng 48:490-500 (1995)), hemoglobin can be lyophilized without oxidation with the use of certain sugars. Results from our model using AAPH suggest that protein with polyols may be left unprotected when faced with alkylperoxides.

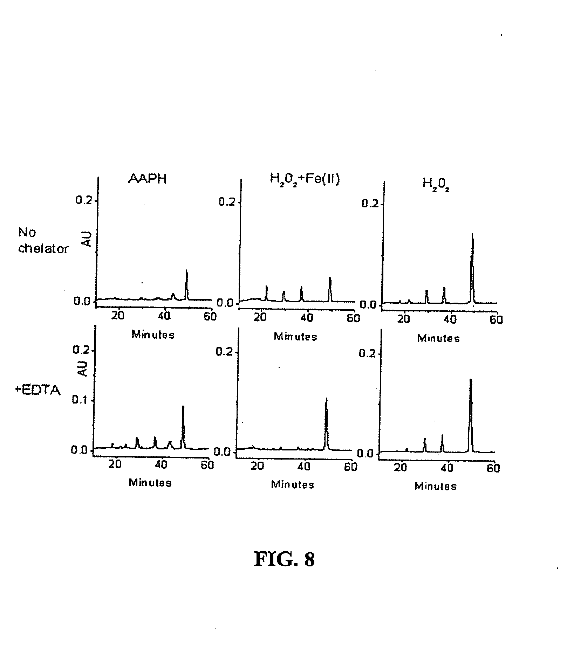

**EDTA:** As shown in Figure 8, EDTA completely protected PTH when it was stressed by the H₂O₂/Fe(II). In this instance, EDTA mitigated not just the generation of free radical, but also the oxidative effect of the H2O2. EDTA did not protect PTH when it was stressed by H2O2 alone. EDTA seemed to exacerbate AAPH oxidation, given that there were abundant Met[O]- and Trp[O]-PTH peaks. Reports of the effect of EDTA or other metal chelators (such as EGTA) have been mixed. The metal chelators may enhance or inhibit a metal-catalyzed reaction. One may generalize and say that EDTA, because it sequester copper effectively, inhibits copper-catalyzed reactions. Because it cannot cover all five valences on iron, the EDTA-iron complex sometimes is very reactive. It is unknown why more oxidation was observed when EDTA was added to a reaction mixture of PTH and AAPH, but such an investigation is beyond the scope of this study.

Frequently described in literature, oxidation of Met, Trp or His residues *in vivo* has been attributed to metal catalyzed oxidation (MCO). In the present experiment, PTH oxidized by AAPH with no added metal generated significant amount of Met and Trp, suggesting that neither Met nor Trp oxidation depends solely on metal catalysis.

**Free tryptophan:** In the literature, many substances have been cited as scavengers for free radicals. Thiourea, methanol and uric acid, are examples, however, they are not suitable for use in protein formulation. In addition, butylated hydroxyl-anisol (BHA) and - toluene (BHT) are radical chain reaction terminators that are quite effective in quenching radicals from lipids. Because of their low water solubility, they are not suitable for aqueous formulation of proteins. With proper amount of surfactant, BHA and BHT may be introduced to an aqueous formulation to offer some oxidative protection.

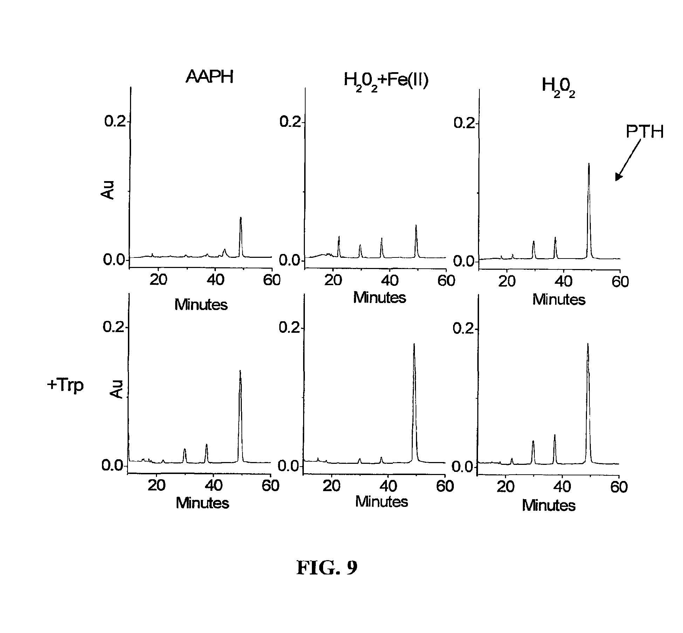

The use of free Trp as an anti-oxidant in parenteral formulation has not been mentioned in the literature. Akin to the use of free Met, it may protect PTH against oxidation. Figure 9 shows that free Trp offers good protection from oxidation of the Trp residue in PTH when PTH is stressed by AAPH or the Fenton reaction. Met[O]-PTH peaks were prominent in the case of AAPH stress and much less so in the case of the Fenton reaction. Free Trp alone offered no protection against oxidative stress by H₂O₂.

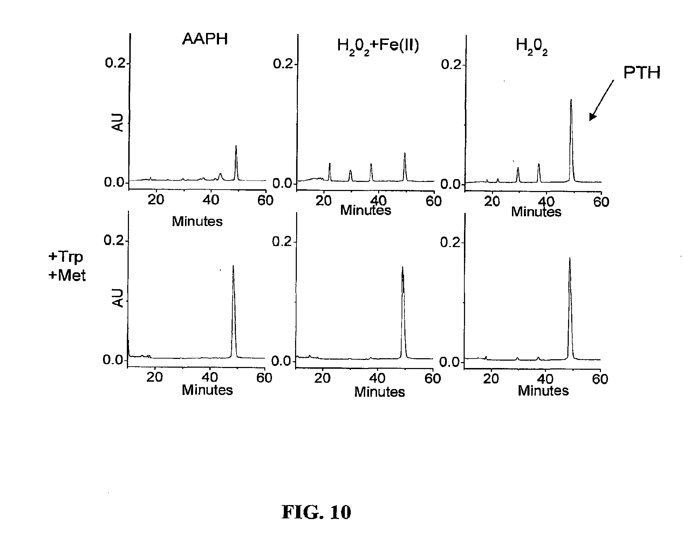

**Combination of tryptophan and methionine:** This combination provided nearly complete protection of PTH under all three oxidative conditions (Figure 10). One can surmise that free Trp and Met will counteract the effect of alkylperoxides and H₂O₂, respectively. When Trp and Met are used in combination, a protein formulation should be able to withstand assault by all three mechanisms shown in Figure 1, as well as light-induced oxidation.

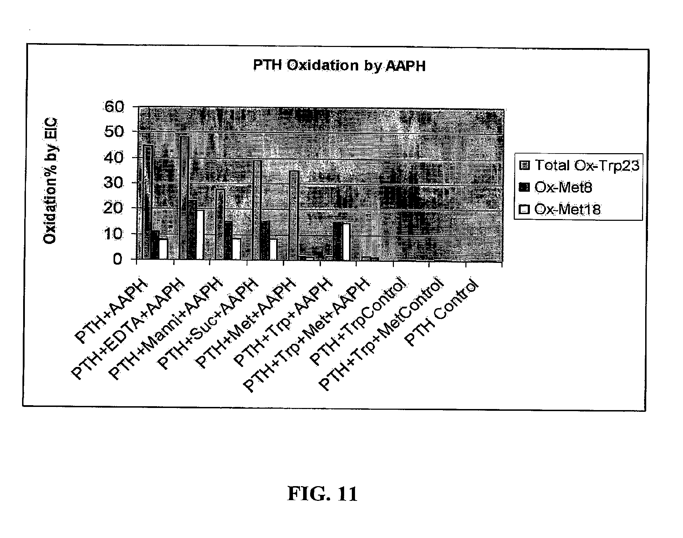

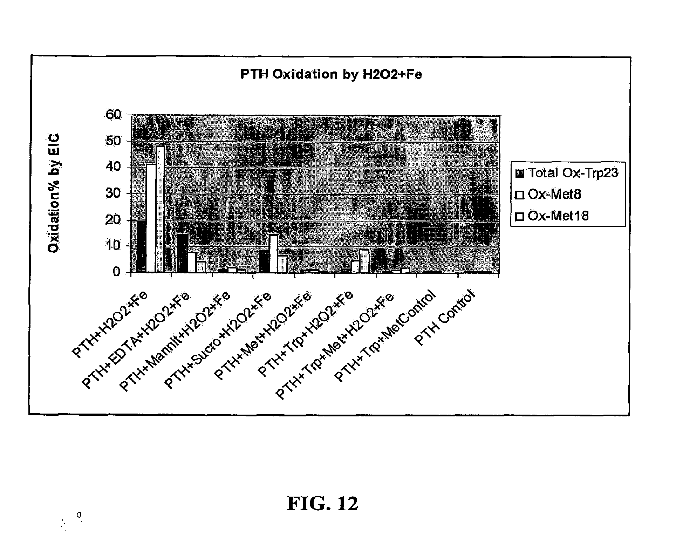

The analysis described above was derived from qualitative examination of the peaks on the respective rp-HPLC chromatograms. Samples oxidized by AAPH and Fenton reaction were subjected to further quantitative and residue-specific analysis by tryptic peptide mapping and LC/MS/MS characterization. According to the relative quantification results obtained by integrating the corresponding EIC (extracted ion chromatogram) of mass signals, free Met alone suppressed the Met oxidation significantly and free Trp alone suppressed the Trp oxidation significantly (Figure 11). The combination of Trp and Met provided the most effective protection against oxidation by AAPH (Figure 11) or the Fenton reaction (Figure 12).

### Site-specific Metal Catalyzed Oxidation:

Trp 50 oxidation of the anti-VEGF antibody was first noted when a qualification lot showed higher degree of main peak loss when stored at 30°C for one month. Autocatalytic oxidation on Trp 50 was later shown to be caused by poorly-handled Tween 20, which resulted in main peak loss and increase of basic peaks in IEC analysis. Further study showed that H₂O₂ did not cause the anti-VEGF antibody oxidation and thus resulted in no change to the IEC chromatographic pattern. Addition of EDTA as a stabilizer and mutation of nearby His resulted in improved stability of the anti-VEGF antibodies with respect to Trp oxidation. Based on these data, it is possible that anti-VEGF antibody oxidation is a site-specific, metal catalyzed reaction.

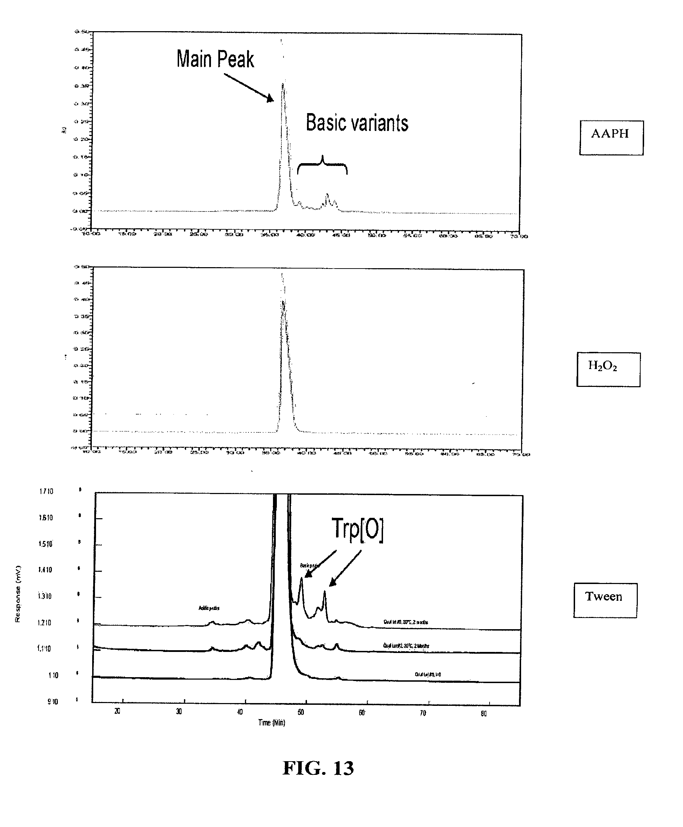

Addition of AAPH to the anti-VEGF antibody solution also produced additional peaks in the basic region of IEC chromatogram, these peaks represented free radical generated anti-VEGF antibody degradants as exhibited by the problematic qualification lot (Figure13). Although these peaks of degraded protein are in comparable position of the degraded peaks from Qualification lots, the profiles are not identical. Further analysis is required to confirm whether these peaks are related to Trp oxidation.

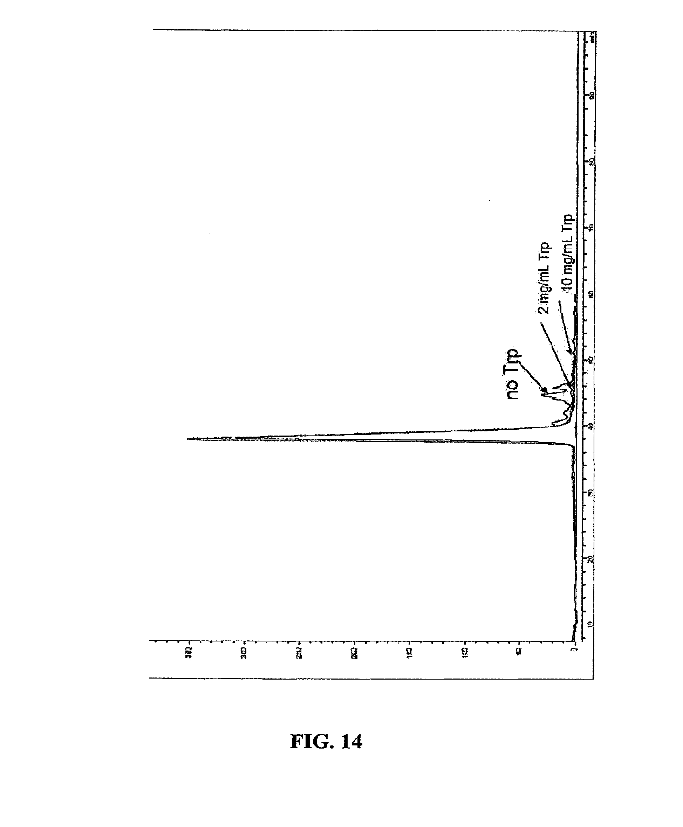

The addition of free tryptophan to the formulation offered an excellent protection against AAPH, as evidenced by the absence of basic peaks in IEC chromatograms (Figure 14). Because Met residues in the anti-VEGF antibody cannot be oxidized by H₂O₂, it is reasonable to expect that free Met would not be needed for anti-VEGF antibodies.

### Anti-CD11a antibody, where no Trp oxidation is expected:

Oxidation of Met residues in anti-CD11a antibodies has been extensively studied. The anti-CD11a antibody has four reactive methionines - Met 256, Met 432, Met 362 and Met 50 (heavy chain). Table 3 shows that H₂O₂, tBHP, and thermal stress caused oxidation of Met 256 and, to a lesser extent, Met 50. In the presence of metal (presumably dissolved metal from stainless steel tank) oxidation at Met 50 increased to a level comparable to that of Met 256. Oxidation was measured by Lys-C peptide mapping. Differences in the order of reactivity were dependent on the type of oxidation stress applied.

**Table 3**

| | **Oxidation Condition** | **Order of Methionine Oxidation** (Most reactive ---> Least reactive) |
|---|---|---|
| **1** | **tBHP** | Met 256 > Met 432 > Met 362 > *Met 50* |
| **2** | **Light** | Met 256 > Met 432 (No other oxidation) |
| **3** | **Thermal** | Met 256 > Met 432 ≈ Met 50 > Met 362 |
| **4** | **Metal Catalyzed** | Met 256 ≈ ***Met 50*** > Met 432 > Met 362 |
| **5** | **H2O2** | Met 256 > Met 432 > *Met 50* |

When AAPH was added to anti-CD11a antibodies, the level of Met 50 increased, suggesting that reactions in a stainless steel tank and the reaction with AAPH are comparable, both resulting in increased oxidation at Met 50 (Table 4). It can be assumed that Met50 is the site of oxidation when metal or free radicals are involved. It is very important to note that under these conditions no Trp, His or Tyr residues were oxidized. However, when denaturant is used, Trp can be oxidized in various places.

**Table 4**

| | % Oxidized | | | |
|---|---|---|---|---|
| | | | | |

| | HC-M256 | HC-M50 | HC-M362 | HC-M35 |
|---|---|---|---|---|
| t=0 | 5.3 | 1.6 | 0.6 | 0.5 |
| t=8hr | 14.3 | 8.2 | 1.2 | 2.3 |
| t=24hr | 31.6 | 20 | 3.2 | 4.1 |
| | | | | |
| Reference | 3 | 0.7 | 1 | 0.4 |

When the ratio of oxidant to protein was increased and when the protein's tertiary structure was perturbed by the addition of guanidine, oxidation of Trp residues by AAPH was abundant. Moreover, under these conditions, no preferential oxidation of Met50 was observed (data not shown).These results further support the use of AAPH as a model oxidizing agent. AAPH reliably causes Trp oxidation when the proper oxidant/protein ratio is used. AAPH does not cause Trp oxidation when normal handling conditions do not generate Trp oxidation. as is the case with anti-CD11a antibody.

### CONCLUSION

In this report we demonstrated that AAPH is a good model oxidant that can oxidize Trp residues in a protein. It is prudent to use H₂O₂, H₂O₂ plus iron and AAPH (Figure1) together to screen stabilizers for protection against oxidation of Met and Trp residues. This system allows for improved simulation all possible oxidative route that may happen during manufacturing and storage of protein pharmaceuticals. Anti-VEGF antibody degradation was separately demonstrated as a metal catalyzed oxidation.

We also demonstrated for the first time that free Trp, when added to protein-containing formulations, effectively blocked the oxidation of tryptophan residues. Under oxidative stress simulated by using AAPH, tryptophan effectively blocked the oxidative reaction in The anti-VEGF antibody. This result suggests that free Trp is effective against site-specific metal-catalyzed oxidation. To ensure that all vulnerable amino acid residues such as Met, Trp, and possibly Hi are protected, a combination of Met and Trp should be considered.

## Claims

1. A pharmaceutical formulation comprising a protein, free methionine and one or more compounds capable of preventing the oxidation of aromatic amino acid residues within said protein, wherein said compounds comprise one or more vitamins or vitamin derivatives.

2. The formulation of Claim 1 wherein said protein is selected from the group consisting of peptides, proteins, antibodies and analogs thereof.

3. The formulation of Claim 2 wherein said antibody is a monoclonal antibody.

4. The formulation of Claims 1-3 wherein said protein has anti-angiogenic properties.

5. The formulation of Claims 1-4 wherein said protein is an anti-VEGF monoclonal antibody.

6. The formulation of Claims 1-3 wherein said protein is an anti-CD20 monoclonal antibody.

7. The formulation of Claims 1-3 wherein said protein is an anti-CD11a monoclonal antibody.

8. The formulation of Claims 1-7 wherein said protein is susceptible to oxidation.

9. The formulation of Claim 1-7 wherein said protein is susceptible to aggregation.

10. The formulation of Claims 1-9 wherein the aromatic amino acid residues within said protein are selected from the group consisting of tryptophan, histidine, tyrosine and phenylalanine.

11. The formulation of Claims 1-10 which is aqueous.

12. The formulation of Claim 1-11 wherein said compounds capable of preventing oxidation are suitable for parenteral injection.

13. The formulation of Claim 1-12 wherein said compounds do not contribute pharmacological effects.

14. The formulation of Claims 1-13 wherein said vitamin or vitamin derivative is 6-hydroxy-2,5,7,8-tetramethylchroman-2-carboxylic acid.

15. The formulation of Claims 1-13 wherein said vitamin or vitamin derivative is pyridoxine.

16. The formulation of Claims 1-15 wherein the antioxidant vitamin or vitamin derivative is present in the formulation in an amount ranging from about 0.1 - 10 mg/ml.

17. The formulation of Claims 1-16 further containing a surfactant.

18. The formulation of Claims 1-17 further containing mannitol.

19. The formulation of Claims 1-18 for use in preventing or treating a disease or disorder.

20. A method of making a pharmaceutical formulation comprising preparing the formulation of Claims 1-18 and evaluating physical stability, chemical stability, or biological activity of the protein in the formulation.

21. A method of stabilizing a pharmaceutical composition of a protein which comprises adding methionine and one or more compounds to said composition in an amount sufficient to inhibit oxidation of aromatic amino acid residues within said protein, wherein said compounds comprise one or more vitamins or vitamin derivatives.

22. A method of making a pharmaceutical formulation comprising adding an amount of a surfactant to a protein composition and an amount of a compound sufficient to negate the oxidative species generated from the degradation of said surfactant, wherein said compound comprises one or more vitamins or vitamin derivatives.

23. A method of preventing the oxidation of aromatic amino acid residues within a susceptible protein which comprises adding methionine in combination with one or more compounds selected from vitamins or their derivatives.


SEQUENCE LISTING

<110>  F. Hoffmann-La Roche AG

<120>  Compositions and Mehtods for the Prevention of Oxidative
       Degradation of Proteins

<130>  27001

<150>  US 61/095,878
<151>  2008-09-10

<160>  2

<170>  PatentIn version 3.5

<210>  1
<211>  34
<212>  PRT
<213>  Homo sapiens

<400>  1

Ser Val Ser Glu Ile Gln Leu Met His Asn Leu Gly Lys His Leu Asn
1               5                   10                  15

Ser Met Glu Arg Val Glu Trp Leu Arg Lys Lys Leu Gln Asp Val His
            20                  25                  30

Asn Phe

<210>  2
<211>  5
<212>  PRT
<213>  Homo sapiens

<400>  2

Val Gly Trp Leu Arg
1               5